(19) Europäisches Patentamt
European Patent Office
Office européen des brevets

(11) Numéro de publication: **0 414 686 B1**

(12) **FASCICULE DE BREVET EUROPEEN**

(45) Date de publication de fascicule du brevet: **22.09.93**

(51) Int. Cl.[5]: **C07C 22/04**, C07C 25/22, C07C 35/52, C07C 43/225

(21) Numéro de dépôt: **89900254.7**

(22) Date de dépôt: **24.11.88**

(86) Numéro de dépôt internationale : **PCT/FR88/00576**

(87) Numéro de publication internationale : **WO 89/04819 (01.06.89 89/12)**

# (54) DERIVES DE TRIFLUOROMETHYL-1-TETRALINES, LEUR PREPARATION ET LEUR APPLICATION POUR LA SYNTHESE DE COMPOSES PRESENTANT DES PROPRIETES THERAPEUTIOUES.

(30) Priorité: **26.11.87 FR 8716435**

(43) Date de publication de la demande: **06.03.91 Bulletin 91/10**

(45) Mention de la délivrance du brevet: **22.09.93 Bulletin 93/38**

(84) Etats contractants désignés: **AT BE CH DE FR GB IT LI LU NL SE**

(56) Documents cités:

**JOURNAL OF ORGANIC CHEMISTRY, vol. 53, no. 4, 19 février 1988, American Chemical Society, Easton, USA; D. BONNET-DELPON et al: "Alpha-Trifluoromethyl-destabilized cations. A route to 1-(trifluoromethyl)tetralins by trifluoroacetolysis of 5-aryl-1,1,1-trifluoropentan-2-ols and derivatives", pages 754-759**

(73) Titulaire: **LABORATOIRES LUCIEN**
**3, rue des Ecoles**
**F-92700 Colombes(FR)**

(72) Inventeur: **BONNET-DELPON, Danièle**
**125, rue de la Réunion**
**F-75020 Paris(FR)**
Inventeur: **CAMBILLAU, Christian**
**26, boulevard du Plateau**
**Le Haut-Verduron F-13015 Marseille(FR)**
Inventeur: **CHARPENTIER-MORIZE, Micheline**
**10, allée Diane-de-Poitiers**
**F-75019 Paris(FR)**

(74) Mandataire: **Poidatz, Emmanuel**
**Cabinet M. SABATIER 83, Avenue Foch**
**F-75116 Paris (FR)**

**EP 0 414 686 B1**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).

JOURNAL OF ORGANIC CHEMISTRY, vol. 53, no. 4, 19 février 1988, American Chemical Society, Easton, USA; D. BONNET-DELPON et al: "Intramolecular Friedel-Crafts alkylation and chloroalkylation of 5-aryl-1,1,1-trifluoropentan-2-ones. A route to (trifluoromethyl)dihydronaphthalenes and (trifluoromethyl)tetrahydronaphthalenes", pages 759-762

## Description

La présente invention concerne des dérivés de trifluorométhyl-1 tétralines, leurs procédés de préparation et leur application pour la synthèse de composés présentant des propriétés thérapeutiques.

L'introduction d'un groupe trifluorométhyl sur un carbone aliphatique demeure une opération difficile à réaliser. Malgré les nombreux travaux entrepris, aucune technique satisfaisante de trifluorométhylation de cycle aliphatique n'a été trouvée jusqu'à ce jour.

La demanderesse a maintenant mis au point de nouvelles méthodes permettant la préparation de tétralines possédant un groupe trifluorométhyl sur un carbone alicylique.

Les composés selon la présente invention sont notamment utiles comme composés de départ pour la synthèse d'aminotétralines trifluorométhylées présentant des propriétés thérapeutiques très intéressantes.

Ces dérivés de trifluorométhyl-1 tétralines selon l'invention répondent à la formule générale :

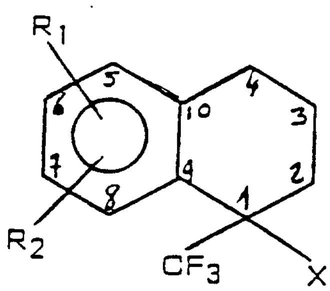

(I)

dans laquelle :

- X représente un atome d'hydrogène, un halogène, un groupe hydroxy, un groupe alcoxy en $C_1$ à $C_8$, ou un noyau aromatique éventuellement substitué;
- $R_1$ représente un atome d'hydrogène ou un halogène, un groupe hydroxy, un groupe alcoxy en $C_1$ à $C_8$, un groupe alkyle linéaire ou ramifié en $C_1$ à $C_4$, éventuellement halogéné, occupant l'une des positions 5, 6 ou 7 ou encore un groupe méthylénedioxy occupant les positions 5 et 6 ou 6 et 7;
- $R_2$ représente un atome d'hydrogène ou un halogène, un groupe hydroxy, un groupe alcoxy en $C_1$ à $C_8$, un groupe alkyle linéaire ou ramifié en $C_1$ à $C_4$ éventuellement halogéné, occupant l'une des autres positions 5,6 ou 7.

Parmi les composés de formule I définis ci-dessus une classe préférée de composés comprend les dérivés dans lesquels X représente un noyau aromatique, notamment phényl, naphtyl, ou thienyl, le noyau phényl étant préféré, pouvant porter un à deux substituants choisis parmi halogéno, hydroxy, alcoxy en $C_1$ à $C_8$ trifluorométhyl ou alkyle linéaire ou ramifié en $C_1$ à $C_4$ éventuellement halogéné.

A titre d'halogène on préfère particulièrement le chlore ou la fluor.

Lorsque les composés de formule I peuvent coexister sous forme d'isomères optiques, l'invention couvre toutes ces formes d'isomères y compris les racémiques et formes individuelles. A titre d'exemples non limitatifs de composés de formule I selon la présente invention, on peut citer les dérivés du tableau 1 suivant :

| Composé n° | X | $R_1$ | $R_2$ |
|---|---|---|---|
| 1 | 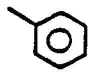 | H | H |
| 2 | 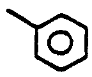 | $-CH_3$ en position 7 | H |
| 3 | 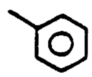 | $-OCH_3$ en position 7 | H |
| 4 | 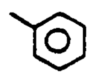 | - Cl en position 7 | H |
| 5 | 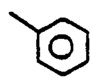 | - Cl en position 6 | H |
| 6 | 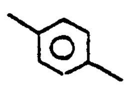 $OCH_3$ | H | H |
| 7 | 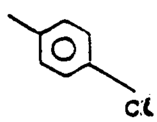 Cl | H | H |

| Composé n° | X | $R_1$ | $R_2$ |
|---|---|---|---|
| 8 | 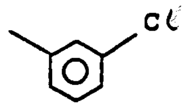 | H | H |
| 9 | H | H | H |
| 10 | H | - $CH_3$ en position 7 | H |
| 11 | - OH | H | H |
| 12 | - Cl | H | H |
| 13 | 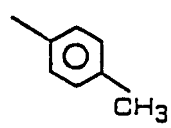 | H | H |
| 14 | - Cl | $CH_3$ en position 7 | H |
| 15 | - Cl | - $OCH_3$ en position 7 | H |
| 16 | - Cl | - Cl en position 7 | H |

| Composé n° | X | $R_1$ | $R_2$ |
|---|---|---|---|
| 17 | OH | - Cl en position 7 | H |
| 18 | - Cl | - Cl en position 6 | H |
| 19 | 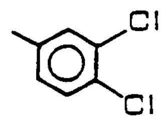 | H | H |
| 20 | 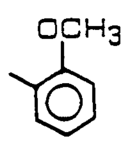 | H | H |
| 21 | 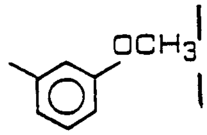 | H | H |

| Composé n° | X | $R_1$ | $R_2$ |
|---|---|---|---|
| 22 | 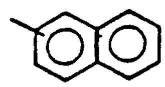 | H | H |
| 23 | 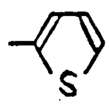 | H | H |

Présentation de la figure :

L'unique figure représente le schéma réactionnel général mis en oeuvre dans le cadre de la présente invention.

Description des modes de réalisation de l'invention :

Les composés de la présente invention peuvent être préparés par les divers procédés définis ci-après à partir des aryl-5-trifluoro-1,1,1-pentane-one-2 connues de formule suivante :

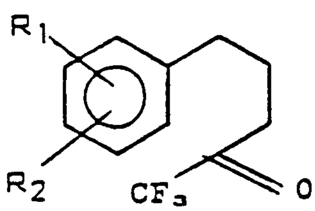

(II)

dans laquelle $R_1$ et $R_2$ ont la même signification que dans la formule I.

Les composés de formule I peuvent être préparés par réaction de Friedel et Crafts intramoléculaire d'une cétone de formule II, dans un solvant en présence d'un acide de Lewis, le radical X pouvant provenir soit de l'acide de Lewis, soit du solvant.

A titre d'acide de Lewis on préfère particulièrement les acides de Lewis aluminiques et $TiCl_4$, avec lesquels le rendement des réactions est meilleur.

Les composés de formule I dans laquelle X est différent d'un noyau aromatique peuvent être préparés en utilisant comme solvant les solvants inertes habituels d'une réaction de Friedel et Crafts, tel que $CH_2Cl_2$.

Les composés de formule I, dans laquelle X représente un noyau aromatique éventuellement substitué peuvent être préparés par réaction de Friedel et Crafts intramoléculaire d'une cétone de formule II dans un solvant qui est un hydrocarbure aromatique correspondant au radical X et en présence d'un acide de Lewis, $AlCl_3$ étant préféré.

L'étude du mécanisme de la cyclisation des cétones de formule II selon une réaction de Friedel et Crafts intramoléculaire a permis d'identifier le schéma réactionnel général de la figure 1. Les acides de

Lewis permettent la cyclisation des cétones de formule II selon une réaction de Friedel et Crafts intramoléculaire, l'hydrocarbure aromatique utilisé comme solvant pouvant participer à la réaction en apportant le radical X pour obtenir les composés de formule I dans lequel X représente un noyau aromatique éventuellement substitué.

Le schéma réactionnel général montre que selon les conditions des réactions, des composés de formule V ou VI, notamment, peuvent être formés soit conjointement avec un composé de formule I, soit isolément.

Ainsi les dérivés du trifluorométhyl-1-dihydronaphtaléne de formule V sont des produits intermédiaires bien intéressants susceptibles d'être obtenus conjointement avec les composés de formule I dans laquelle X représente un halogène.

Les exemples ci-après, donnés à titre d'illustration des procédés de préparation de composés de formule I selon une réaction de Friedel et Crafts intramoléculaire d'une cétone de formule II, mettront en évidence d'autres avantages et modes de réalisation du présent procédé.

A. Préparation des Aryl-5-trifluoro-1,1,1 pentane-one-2 de formule 2 :

Protocole expérimental : une solution de bromure d'éthyl magnésium (0,2 mole) dans de l'éther anhydre (100 ml) est ajoutée goutte à goutte, sous azote, à une solution d'acide trifluoroacétique (0,2 mole) dans de l'éther anhydre, sous agitation à -5 ° C. Le mélange réactionnel est laissé à température de la pièce et produit le sel de bromure de magnésium de l'acide trifluoroacétique.

Une solution de l'aryl-3-bromo-1-propane choisi (0,25 mole) dans le l'éther anhydre (150 ml) est ajoutée goutte à goutte, sous agitation, à du magnésium dans de l'éther anhydre (10 ml) à un taux ajusté pour maintenir un reflux stable. Après deux heures, on ajoute goutte à goutte, à la solution de Grignard obtenue, sous azote, une suspension de sel d'acide trifluoroacétique de -5 ° C. Après agitation d'une heure à température de la pièce et reflux d'une heure, le mélange est refroidi et hydrolisé par 100 ml d'acide chlorhydrique (10 %) ajouté goutte à goutte a 0 ° C. La couche aqueuse est extraite avec de l'éther. Les couches organiques combinées sont lavées avec de la saumure, séchées (Mg So4), concentrées et distillées.

Les aryl-3-bromo-1-propane utilisés pour la préparation des aryl-5-trifluoro-1,1,1-pentane-one-2 correspondants sont d'une manière générale disponibles dans le commerce.

Exemples :

1) Préparation du phényl-5-trifluoro-1,1,1-pentane-one-2 :
de formule II

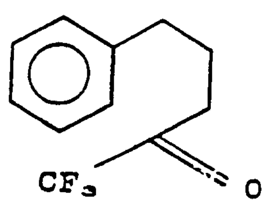

selon le protocole expérimental en utilisant le phényl-3-bromo-1-propane; le rendement de la réaction est de 48%, la température d'ébullition est de 90 ° C sous une pression de 10 mmHg.

2) Préparation du paraméthylphényl-5-trifluoro-1,1,1-pentane-one-2 : de formule II

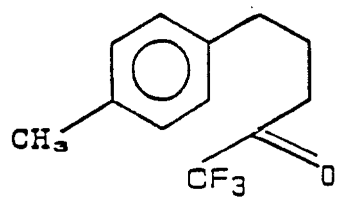

selon le protocole expérimental en utilisant le phénylparaméthyl-3-bromo-1-propane; le rendement de la réaction est de 45 % après purification par chromatographie ($SiO_2$, pentane/$Et_2O$ 10%).

3) Préparation du paraméthoxyphényl-5-trifluoro-1,1,1-pentane-one-2 : de formule II

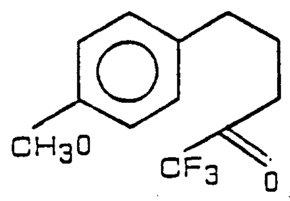

selon le protocole expérimental en utilisant le phénylparamathoxy-3-bromo-1-propane; le rendement de la réaction est de 48% après purification par chromatographie ($SiO_2$, pentane /$Et_2O$ 10%), la température d'ébullition est de 82 °C sous une pression de 0,5 mmHg.

4) Préparation du parachlorophényl-5-trifluoro-1,1,1-pentane-one-2 : de formule II

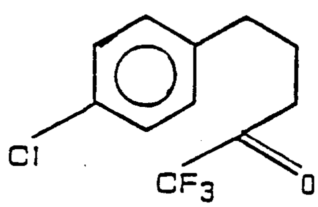

et du métachlorophényl-5-trifluoro-1,1,1-pentane-one-2 de formule II

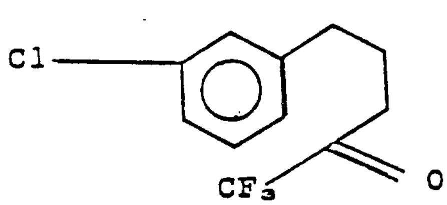

selon le protocole expérimental en utilisant le phénylparachloro-3-bromo-1-propane et le phénylmétachloro-3-bromo-1-propane. Toutefois, ces produits n'étant pas disponibles dans le commerce, il est nécessaire de les préparer préalablement à partir :

- du phénylparachloro-5-propanol, et
- du phénylmétachloro-5-propanol.

Préparation du chlorophényl-3-propanol (méta ou para) :

Une solution' d'éther et de (méta ou para) chlorobenzyl magnésium chloride préparée à partir de 80 g (0,5 mole) de (méta ou para) -chlorobenzyl chloride et de 13 g (0,53 mmole) de magnésium dans 500 ml d'éther est refroidie dans un bain de glace, et un double excès d'éthylène oxyde dans 100 ml d'éther est ajouté à un taux modéré. De l'acide sulfurique dilué (10%) est ajouté et les couches sont séparées. La couche organique est lavée avec de l'eau, séchée sur du sulfate de magnésium et concentrée, puis l'huile est distillée. Pour le métachlorophényl-3-propanol, la température d'ébullition est de 115°C sous une pression de 0,4 mmHg. Le rendement de la réaction est de 63%.

Pour le parachlorophényl-3-propanol, la température d'ébullition est de 98°C sous une pression de 0,1 mmHg, le rendement de la réaction est de 56%.

Préparation du chlorophényl-3-bromo-1-propane (méta ou para):

Du tribromure de phosphore (27g, 0,1 mole) est ajouté à 34 g (0,2 mole) de chlorophényl-3-propanol (méta ou para) refroidi à 5°C. Amené à la température de la pièce, le mélange est porté à 100°C pendant une heure. Après refroidissement, de la glace est ajoutée et la couche organique est extraite avec du pentane. La couche organique est lavée jusqu'à neutralité et séchée sur du sulfate de magnésium. Le produit brut est filtré sur de l'alumine et la distillation de l'éluat fournit un produit pur avec un rendement de 65% (température d'ébullition 92°C sous une pression de 0,3 mmHg) pour le parachlorophényl-3-bromo-1-propane et avec un rendement de 77% (température d'ébullition 75°C sous une pression de 0, 1 mmHg) pour le métachlorophényl-3-bromo-1-pentane.

En utilisant le parachlorophényl-3-bromo-1-propane, on obtient le parachlorophényl-5-trifluoro-1-pentane-one-2 avec un rendement de 46% (la température d'ébullition étant de 86°C sous une pression de 0,3 mmHg).

En utilisant le métachlorophényl-3-bromo-1-propane on obtient le métachlorophényl-5-trifluoro-1-pentane-one-2 avec un rendement de 43% (la température d'ébullition étant de 72°C sous une pression de 0,2 mmHg).

B/ Cyclisation des Aryl-5-trifluoro-1,1,1 pentane-one-2 de formule II dans un solvant en présence d'un acide de Lewis:

Protocole expérimental : les réactions sont effectuées dans un solvant sec sous courant de gaz neutre tel que l'argon, avec un volume réactionnel initial ajusté pour disposer environ d'une solution 0.3 molaire en ketone de formule II.

La solution est refroidie à la température désirée et un équivalent d'acide de Lewis est ajouté goutte à goutte ou par petites quantités sous agitation. Lorsque les produits de départ ont disparu, le mélange est hydrolysé avec un volume égal d'eau. La couche organique est alors lavée deux fois avec de la saumure séchée sur du $MgSO_4$ anhydre et concentrée par évaporation rotative. Le produit pur est ensuite purifié par chromatographie sur colonne ($SiO_2$ neutre, maille 70-230) en utilisant un éluant pentane/éther.

Exemples :

1) Cyclisation du phényl-5-trifluoro-1,1,1-pentane-one-2:

($R_1$ et $R_2$ représentent un atome d'hydrogène dans la formule II).

a) Avec $SbF_5$ dans $CH_2Cl_2$ :

A une solution de 432 mg (2 mmoles) de phényl-5-trifluoro-1, 1,1-pentane-one-2 dans 8 ml de $CH_2Cl_2$, on ajoute à une température de 0°C 2 ml d'une solution 1 molaire de $SbF_5$ dans $CH_2Cl_2$. Après trois heures, la solution est neutralisée avec $NaHCO_3$, lavée avec de l'eau, séchée et évaporée, pour donner 195 mg (soit un rendement de 50%) d'un mélange des composés suivants :

- 33% d'un composé de formule V dans laquelle $R_1$ et $R_2$ représentent un atome d'hydrogène;
- 35% d'un composé de formule I qui est le trifluorométhyl-1-tétrahydronaphtalène-1,2,3,4, (composé n° 9 du tableau 1);
- 32% d'un composé de formule VI dans laquelle $R_1$ et $R_2$ représentent un atome d'hydrogène.

b) Avec $AlCl_3$ dans $CH_2Cl_2$ :

216 mg (1 mmole) de phényl-5-trifluoro-1,1,1-pentane-one-2 dans 3 ml de $CH_2Cl_2$ traités à une température de 20 °C avec 133 mg (1 mmole) de $AlCl_3$, pendant une durée de 1h30, donnent 164 mg (soit un rendement de 70 %) de trifluorométhyl-1-chloro-tétrahydronaphtalène-1,2,3,4 (composé n° 12 du tableau 1).

Si la réaction est maintenue pendant 5 heures, le même composé de formule I est obtenu mais avec un rendement de 60 %.

c) Avec $AlCl_3$ dans du benzène :

432 mg (2 mmoles) de phényl-5-trifluoro-1,1,1-pentane-one-2 dans 7 ml de benzène traités à une température de 5 °C avec 266 mg (2 mmoles) de $AlCl_3$, pendant une durée de 2 heures, donnent 300 mg, soit un rendement de 54 %, du composé n°1 du tableau 1. Si la réaction est maintenue pendant un court instant, on obtient un mélange du composé n° 12 et du composé n° 1 du tableau 1.

d) Avec $AlCl_3$ dans du toluène :

432 mg (2 mmoles) de phényl-5-trifluoro-1,1,1-pentane-2 dans 7 ml de toluène traités à une température de 5 °C avec 266 mg (2 mmoles) de $AlCl_3$ pendant une durée de deux heures, donnent 348 mg (soit un rendement de 60 %) de trifluorométhyl-1, 1,1-tétrahydronaphtalène-1,2,3,4 (composé n° 13 du tableau 1).

e) Avec $AlCl_3$ dans le l'anisole :

432 mg (2 mmoles) de phényl-5-trifluoro-1,1,1-pentane-one-2 dans 7 ml d'anisole traités à une température de 5 °C avec 266 mg (2 mmoles) de $AlCl_3$ pendant une durée de deux heures donnent 459 mg (soit un rendement de 75 %) de trifluorométhyl-1-paraméthoxyphényl-1-tétrahydronaphtalène-1,2,3,4 (composé n° 6 du tableau 1).

f) Avec $TiCl_4$ dans $CH_2Cl_2$ :

540 mg (2,5 mmoles) de phényl-5-trifluoro-1,1,1-pentane-one-2 dans 7 ml de $CH_2Cl_2$ traités à une température de 0 °C avec 475 mg (0,275 ml, 2,5 mmoles) de $TiCl_4$ pendant trois heures donnent 526 mg (soit un rendement de 90 %) du composé n° 12 du tableau 1.

La même réaction effectuée à 20 °C pendant cinq heures donne 25 mg (soit un rendement de 5%) d'un composé de formule V dans laquelle $R_1$ et $R_2$ représentent un atome d'hydrogène et 465 mg (soit un rendement de 80%) du composé n° 12 du tableau 1.

g) Avec $TiCl_4$ dans du benzène :

540 mg (2,5 mmoles) de phényl-5-trifluoro-1,1,1-pentane-one-2 dans 7 ml de benzène traités à une température de 5 °C avec 475 mg (0,275 ml, 2,5 mmoles) de$TiCl_4$ pendant trois heures donnent avec un rendement de 55%, le composé n° 12 du tableau 1.

Quand la réaction est maintenue pendant une durée plus longue ou à une température supérieure, le même produit est obtenu.

h) Avec $TiCl_4$ dans $CH_2Cl_2$ à - 50 °C :

540 mg (2,5 mmoles) de phényl-5-trifluoro-1,1,1-pentane-one-2 dans 7 ml de $CH_4Cl_2$ traités à une température de - 50 °C avec 475 mg (0,275 ml, 2,5 mmoles) de $TiCl_4$ pendant une durée de quarante huit heures, donnent 146 mg (soit un rendement de 25%) du composé n° 12 du tableau 1, 130 mg (soit un rendement de 24%) de la cétone de départ et 248 mg (soit un rendement de 46%) de trifluorométhyl-1-hydroxy-1-tétrahydronaphtalène-1,2,3,4 (composé n° 11 du tableau 1).

2) Cyclisation du phénylparaméthyl-5-trifluoro-1,1,1-pentane-one-2 :

($R_1$ représente un groupe méthyl en position para et $R_2$ représente un atome d'hydrogène dans la formule II).

1 g (4 mmoles) de phénylparaméthyl-5-trifluoro-1,1,1-pentane-one-2 dans 12 ml de $CH_2Cl_2$ traité à une température de 0 °C avec 824 mg (0,477 ml, 4,34 mmoles) de $TiCl_4$ pendant une durée de trois heures donne 865 mg (soit un rendement de 80 %) de trifluorométhyl-1-chloro-1-méthyl-7-tétrahydronaphtalène-1,2,3,4 (composé n° 14 du tableau 1).

3) Cyclisation du paraméthoxyphényl-5-trifluoro-1,1,1-pentane-one-2 :

($R_1$ représente un groupe méthoxy en position para et $R_2$ représente un atome d'hydrogène dans la formule II).

1 g (4,06 mmoles) de phénylparaméthoxy-5-trifluoro-1,1,1-pentane-one-2 dans 12 ml de $CH_2Cl_2$ traité à une température de 0 °C avec 824 mg (0,477 ml, 4,34 mmoles) de $TiCl_4$ pendant une durée de trois heures donne 860 mg (soit un rendement de 80%) de trifluorométhyl-1-chloro-1-méthoxy-7-tétrahydronaphtalène-1,2,3,4 (composé n° 15 du tableau 1).

4) Cyclisation du parachlorophényl-5-trifluoro-1,1,1-pentane-one-2 :

1 g (4 mmoles) de phénylparachloro-5-trifluoro-1,1,1-pentane-one-2 dans 12 ml de $CH_2Cl_2$ traité à une température de 20°C avec 824 mg (0,477 ml, 4,34 mmoles) de $TiCl_4$ pendant une durée de vingt-quatre heures donne 160 mg (soit un rendement de 15%) de trifluorométhyl-1-chloro-1-chloro-7-tétrahydronaphtalène-1,2,3,4 (composé n° 16 du tableau 1), 400 mg (soit un rendement de 40 % de trifluorométhyl-1-hydroxy-1-chloro-7-tétrahrdronaphtalène-1,2,3,4 (composé n° 17 du tableau 1).

5) Cyclisation du phénylmétachloro-5-trifluoro-1,1,1-pentane-one-2 :

500 mg (2 mmoles) de phénylmétachloro-5-trifluoro-1,1,1-pentane-one-2 dans 6 ml de $CH_2Cl_2$ traités à une température de 0°C avec 380 mg (0,22 ml, 2 mmoles) de $TiCl_4$ pendant trois heures donnent 430 mg (soit un rendement de 80 %) de trifluorométhyl-1-chloro-1-chloro-6-tétrahydronaphtalène-1,2,3, 4 (composé n° 18 du tableau 1).

6) Formation du composé n° 1 du tableau 1 à partir du composé n° 12 du tableau 1 :

468 mg (2 mmoles) du composé n° 12 dans 7 ml de benzène traités à une température de 5°C pendant une durée de deux heures avec 266 mg (2 mmoles) de $AlCl_3$ donnent 250 mg (soit un rendement de 45 %) du composé n° 1.

Solvolyse

Les composés de formule I dans laquelle X représente un noyau aromatique éventuellement substitué peuvent également être préparés par solvolyse d'un dérivé d'un aryl-5-trifluoro-1,1,1-pentane-ol-2 de formule III

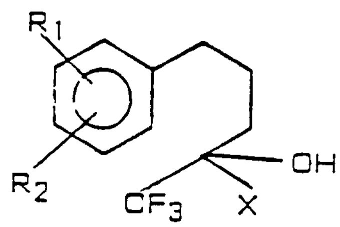

(III)

dans laquelle $R_1$ et $R_2$ ont la même signification que dans la formule I et X représente un noyau aromatique éventuellement substitué.

Les dérivés des aryl-5-trifluoro-1,1,1-pentane-ol-2 (III) peuvent être préparés par des méthodes classiques d'addition d'organométalliques sur les dérivés des aryl-5-trifluoro-1,1,1-pentane-one-2 (II) correspondantes (connues).

La réaction de solvolyse consiste à faire réagir en milieu acide un dérivé de formule III pour obtenir le composé de formule I correspondant.

Le milieu acide peut être constitué d'un acide protique tel que l'acide trifluoroacétique. La solvolyse d'un dérivé de formule III peut alors être effectuée dans un autoclave immergé dans un bain d'huile maintenu à une température comprise entre 120°C et 180°C et pendant une durée comprise entre soixante minutes et deux cent quarante minutes, pour obtenir le composé de formule I correspondant; ce dernier après évaporation de l'acide trifluoroacétique est extrait avec $CH_2Cl_2$, lavé avec $NaHCO_3$ et de l'eau jusqu'à neutralité, séché sur $MgSO_4$, évaporé et purifié par chromatographie ($SiO_2$, pentane-éther : 10%).

Le milieu acide peut également être constitué d'un acide protique tel que l'acide sulfurique, dans un isolant , tel que l'acide trifluoroacétique, le dichlorométhane, le benzène ou le toluène. La solvolyse d'un dérivé de formule III peut alors être effectuée au reflux pendant une durée inférieure à douze heures, pour obtenir le composé de formule I correspondant, ce dérivé pouvant être isolé comme précédemment.

Le milieu acide peut encore être constitué d'un acide de Lewis, $AlCl_3$ étant préféré, dans un solvant tel que le dichlorométhane, le benzène ou le toluène. La solvolyse d'un dérivé de formule III peut alors être effectuée à température ambiante pendant une durée comprise entre deux heures et cinq heures, pour

obtenir le composé de formule I correspondant, ce dernier pouvant être isolé comme précédemment.

Les composés de formule I dans laquelle X représente un atome d'hydrogène peuvent également être préparés par solvolyse d'un dérivé d'un aryl-5-trifluoro-1,1,1-pentane-ol-2 protégé de formule IV

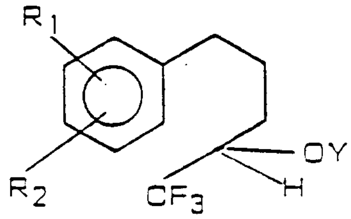

(IV)

dans laquelle :

- $R_1$ et $R_2$ ont la même signification que dans la formule I et OY représente un groupe partant, notamment un groupe tosyle, un groupe triflique, un groupe méthane sulfonique, le groupe triflique étant préféré.

La réaction de solvolyse consiste comme précédemment, à faire réagir en milieu acide un dérivé de formule IV pour obtenir le composé de formule I correspondant.

Le milieu acide est constitué ;

- d'un acide protique tel que l'acide trifluoroacétique et éventuellement de l'acide sulfurique. La solvolyse d'un dérivé de formule IV est effectuée dans un autoclave immergé dans un bain d'huile maintenu à une température comprise entre 120°C et 180°C pendant une durée comprise entre soixante minutes et deux cent quarante minutes pour obtenir le composé de formule I corrrespondant, ce dernier pouvant être isolé comme précédemment.

L'étude poussée du mécanisme de la réaction de solvolyse a montré que la formation d'un composé de formule I par ce procédé pouvait s'effectuer par cyclisation d'un carbocation hautement déstabilisé formé transitoirement à partir du dérivé de formule III correspondant (figure 1).

Dans le cas de la solvolyse d'un dérivé de formule IV, la cyclisation dépend de la nature et de la position de $R_1$ et $R_2$ et peut être interprétée comme un processus k$\Delta$ concerté, impliquant l'assistance nucléophile du groupe $\delta$ phényl.

Ainsi, des rendements très faibles sont observés lorsque l'on effectue la solvolyse des dérivés de formule IV dans laquelle $R_1$ et/ou $R_2$ représentent un halogène ou un groupe méthoxy; des rendements très satisfaisants sont atteints lorsque l'on effectue la solvolyse de dérivés de formule III dans laquelle $R_2$ représente un atome d'hydrogène et $R_1$ représente un atome d'hydrogène ou un groupe méthyl occupant la position 7.

Les exemples ci-après sont donnés à titre d'illustration des procédés de préparation des composés de l'invention par solvolyse.

A) Préparation des dérivés des aryl-5-trifluoro-1,1,1-pentane-ol-2 de formules III et IV :

1) Préparation du phenyl-5-trifluoro-1,1,1-pentane-ol-2 de formule III :

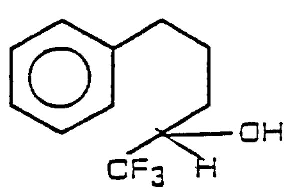

Une solution de phényl-5-trifluoro-1,1,1-pentane-ol-2 (10 mmoles) dans de l'éther anhydre (20 ml) est ajoutée sous agitation à une suspension de $LiAlH_4$ (10 mmoles) dans de l'éther anhydre (20 ml).

Après deux heures à la température de la pièce, la réaction est hydrolysée dans de l'éther et de l'eau. La couche organique est extraite, lavée et séchée sur du sulfate de magnésium. Après évaporation du solvant et distillation, l'alcool pur est obtenu.

Le rendement de la réaction est de 80 %. La température d'ébullition est de 72 ° C sous 0,1 mmHg.

2) Préparation du paraméthylphényl-5-trifluoro-1,1,1-pentane-ol-2 de formule III :

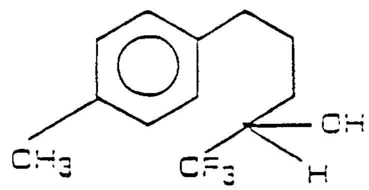

Ce dérivé est obtenu selon le même protocole expérimental que dans l'exemple 1, mais l'on utilise comme cétone de départ la paraméthylphényl-5-trifluoro-1,1,1-pentane-one-2. Le rendement de la réaction est de 95%.

3) Préparation du phényl-5-trifluoro-1,1,1-pentyltriflate-2 de formule IV :

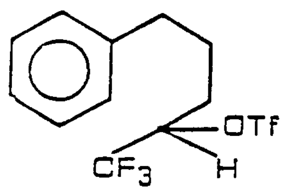

A 0 ° C, sous argon, on ajoute doucement à un mélange agité de pyridine (4 ml) et de $CH_2Cl_2$ (15 ml), tous deux filtrés sur alumine, à l'aide d'une seringue, successivement du triflique anhydre (3 mmoles) et du phényl-5-trifluoro-1,1,1-pentane-ol-2 (2 mmoles).

Le mélange réactionnel est agité pendant quatre heures à 0 ° C et versé sur de l'eau acidifiée (HCl 15%) et de la glace, puis extrait dans $CH_2Cl_2$, lavé jusqu'à nentralite et séché (Mg $SO_4$).

Après soustraction du solvant, le phényl-5-trifluoro-1,1,1-pentyltriflate-2est obtenu. Le rendement de la réaction est de 95%. La température d'ébullition est de 92 ° C sous une pression de 12 mmHg.

4) Préparation du paraméthylphényl-5-trifluoro-1,1,1-pentyltriflate-2 de formule IV :

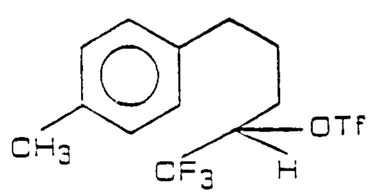

Ce dérivé est obtenu selon le même protocole expérimental que dans l'exemple 3, mais en utilisant comme alcool le paraméthylphényl-5-trifluoro-1,1,1-pentane-ol-2.

Le rendement de la réaction est de 73%.

5) Préparation du paraméthoxyphényl-5-phényl-2-trifluoro-1,1, 1-pentane-ol-2 de formule III :

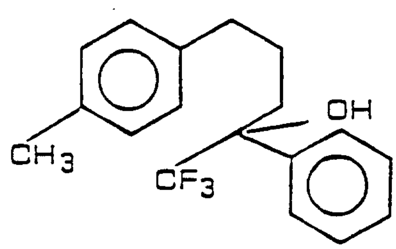

On ajoute sous atmosphère d'argon, un réactif de Grignard à la paraméthoxyphényl-5-trifluoro-1,1,1-pentane-one-2 en utilisant comme solvant de l'éther anhydre. Le mélange réactionnel, après avoir été chauffé et mis au reflux pendant deux heures est refroidi et hydrolysé avec une solution aqueuse de chlorure d'ammonium.

Les techniques usuelles conduisent à l'alcool pur.

Le rendement de la réaction est de 87%, la température de fusion est de 56,3 ° C.

6) Préparation du phényl-5-paraméthoxyphényl-2-trifluoro-1,1, 1-pentane-ol-2 de formule III :

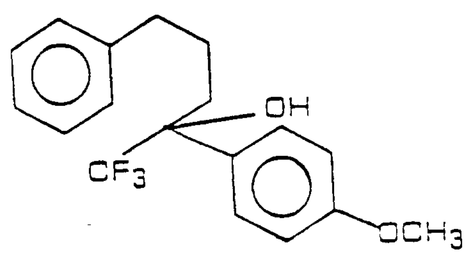

Ce dérivé est obtenu à partir du paraméthoxyphénylmagnésium bromide et de phényl-5-trifluoro-1,1,1-pentane-one-2. Le rendement de la réaction est de 90 % après purification par flash chromatographie.

7) Préparation du métachlorophényl-5-phényl-2-trifluoro-1,1, 1-pentane-ol-2 de formule III :

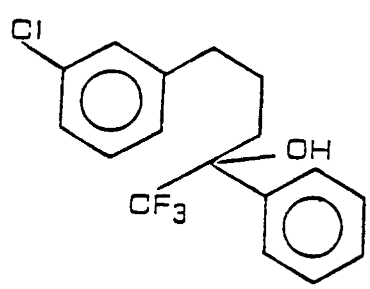

Ce dérivé est obtenu à partir du métachlorophényl-5-trifluorométhyl-1,1,1-pentane-one-2.

Le rendement de la réaction est de 96% après purification par flash chromatrographie.

8) Préparation du phényl-5-métachlorophényl-2-trifluoro-1,1, 1-pentane-ol-2 de formule III :

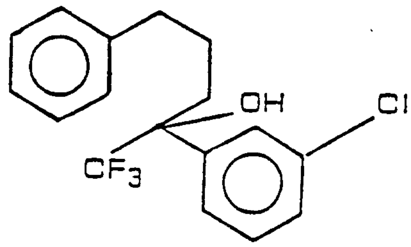

La purification par flash chromatographie conduit à l'alcool pur, le rendement de la réaction est de 62%.

B) Préparation des composés de formule I par solvolyse :

1) Préparation du trifluorométhyl-1-tétrahydronaphtalène-1,2, 3,4 (composé n° 9 du tableau 1) de formule I :

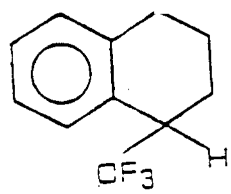

La solvolyse est effectuée dans un autoclave immergé dans un bain d'huile maintenu à 140 °C dans le proportions suivantes :

- 1 mmole de phényl-5-trifluoro-1,1,1-pentyltriflate-2,
- 3 ml d'acide trifluoroacétique et 1 ml d'anhydride trifluoroacétique.

Après quarante-huit heures de réaction, l'acide trifluoroacétique est évaporé et le composé de formule I formé est extrait avec du $CH_2Cl_2$, lavé avec du $NaHCO_3$ et de l'eau jusqu'à neutralité, puis séché sur $MgSO_4$, évaporé et purifié par chromatographie ($SiO_2$, pentane-éther :10%).

350 mg de phényl-5-trifluoro-1,1,1-pentyltriflate-2 sont transformés en 187 mg de trifluorométhyl-1-tétrahydronaphtalène-1,2,3,4 ; soit un rendement de 93%.

2) Préparation du trifluorométhyl-1-méthyl-7-tétrahydronaphtalène-1,2,3,4 (composé n° 10 du tableau 1) de formule I :

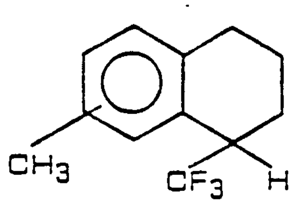

La solvolyse du paraméthylphényl-5-trifluoro-1,1,1-pentyltriflate-2 est effectuée selon le même protocole expérimental que dans l'exemple 1 mais à une température de 160 °C et pendant seize heures.

365 mg de paraméthylphényl-5-trifluoro-1,1,1-pentyltriflate-2 sont transformés en 100 mg de trifluorométhyl-1-méthyl-7-tétra-hydronaphtalène-1,2,3,4 ; soit un rendement de 51%.

3) Préparation du trifluorométhyl-1-phényl-1-tétrahydronaphtalène-1,2,3,4 (composé n° 1 du tableau 1) de formule I :

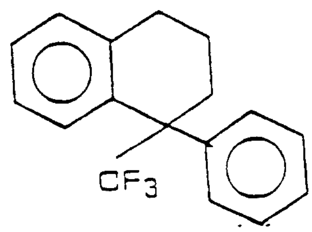

La solvolyse du trifluoro-1,1,1-diphényl-2,5-pentane-ol-2 est effectuée selon le même protocole expérimental que dans l'exemple 1 mais à 120 °C et pendant quinze heures.

294 mg de trifluoro-1,1,1-diphényl-2,5-pentane-ol-2 sont transformés en 240 mg de trifluorométhyl-1-phényl-1-tétrahydronaphtalène-1,2,3,4; soit un rendement de 87%.

4)Le trifluorométhyl-1-phényl-1-tétrahydronaphtalène-1,2,3,4

peut également être préparé par solvolyse au reflux selon le protocole expérimental suivant :
1,5 mmoles (294 mg) de trifluoro-1,1,1-diphényl-2,5-pentane-ol-2 et 25 ml d'une solution d'acide sulfurique $2.10^{-2}$ molaire dans de l'acide trifluoroacétique sont chauffés au reflux pendant une durée de trente minutes pour former après purification 285 mg de trifluorométhyl-1-phényl-1-tétrahydronaphtalène-1,2,3,4; soit un rendement de 85%.

5) Préparation du trifluorométhyl-1-méthyl-7-phényl-1-tétrahydronaphtalène-1,2,3,4 (composé n° 2 du tableau 1) de formule I :

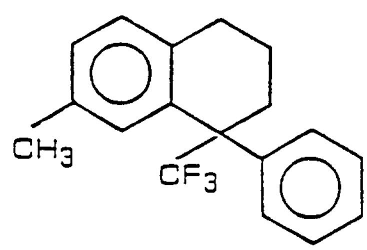

1,5 mmoles (294 mg) de paraméthylphényl-5-phényl-2-trifluoro-1,1,1-pentane-ol-2 et 25 ml d'une solution d'acide sulfurique $2.10^{-2}$ molaire dans de l'acide trifluoroacétique sont chauffés au reflux pendant une durée de trente minutes pour former après purification 285 mg du composé n° 2 du tableau 1; soit un rendement de 85%.

6) Préparation du trifluorométhyl-1-méthoxy-7-phényl-1-tétrahydronaphtalène-1,2,3,4 (composé n° 3 du tableau 1) de formule :

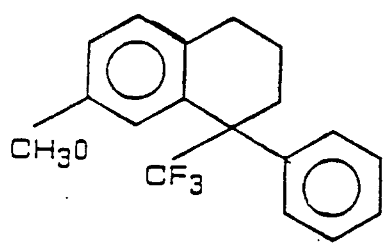

1,5 mmoles (325 mg) de paraméthoxyphényl-5-phényl-2-trifluoro-1,1,1-pentane-ol-2 et 25 ml d'une solution d'acide sulfurique $2.10^{-2}$ m dans de l'acide trifluoroacétique sont chauffés au reflux pendant une durée de trente minutes pour former après purification 306 mg du composé n° 3 du tableau 1 ; soit un rendement de 95 %.

7) Préparation du trifluorométhyl-1-chloro-7-phényl-1-tétrahydronaphtalène-1,2,3,4 (composé n° 4 du tableau 1) de formule I :

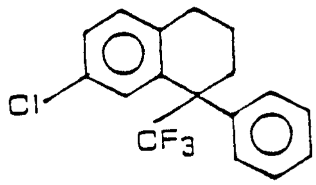

1,5 mmoles (328 mg) de parachlorophényl-5-phényl-2-trifluoro-1,1,1-pentane-ol-2 et 25 ml d'acide sulfurique $2.10^{-2}$ molaire dans de l'acide trifluoroacétique sont chauffés au reflux pendant une durée de trente minutes pour former 280 mg du composé n° 4 du tableau 1; soit un rendement de 90%.

8) Préparation du trifluorométhyl-1-chloro-6-phényl-1-tétrahydronaphtalène-1,2,3,4 (composé n° 5 du tableau 1) de formule I :

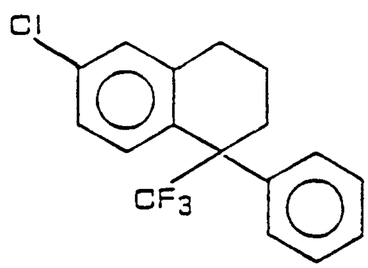

1,5 mmoles (328 mg) de métachlorophényl-5-phényl-2-trifluoro-1,1,1-pentane-ol-2 et 25 ml d'acide sulfurique $2.10^{-2}$ molaire dans de l'acide trifluoroacétique sont chauffés au reflux pendant une durée de trente minutes pour former 280 mg du composé n° 5 du tableau 1; soit un rendement de 90%.

9) Préparation du trifluorométhyl-1-paramethoxyphényl-1-tétrahydronaphtalène-1,2,3,4 (composé n° 6 du tableau 1) de formule I :

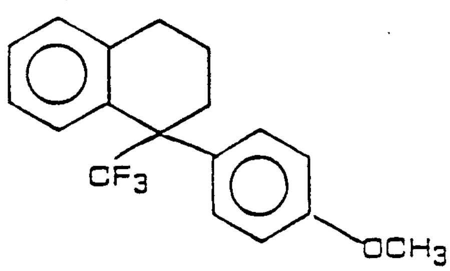

1,5 mmoles (325 mg) de phényl-5-paraméthoxyphényl-2-trifluoro-1,1,1-pentane-ol-2 et 25 ml d'acide sulfurique $2.10^{-2}$ molaire dans de l'acide trifluoroacétique sont chauffés au reflux pendant une durée de cinq minutes pour former après purification 305 mg du composé n° 6 du tableau 1; soit un rendement de 95%.

10) Préparation du trifluoro-1-parachlorophényl-1-tétrahydronaphtalène (composé n° 7 du tableau 1) de formule I :

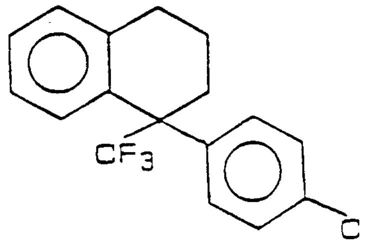

1,5 mmoles (328 mg) de phényl-5-parachlorophényl-2-trifluoro-1,1,1-pentane-ol-2 et 25 ml d'acide sulfurique $2.10^{-2}$ molaire dans de l'acide trifluoroacétique sont chauffés au reflux pendant une durée de trente minutes pour former après purification 263 mg du composé n° 7 du tableau 1 ; soit un rendement de 85 %.

11) Préparation du trifluorométhyl-1-métachlorophényl-1-tétrahydronaphtalène-1,2,3,4 (composé n° 8 du tableau 1) de formule I :

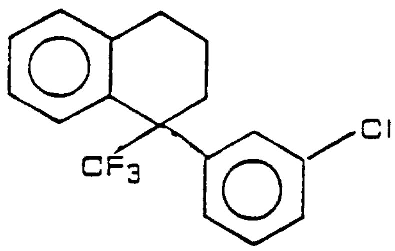

1,5 mmoles (330 mg) de phényl-5-parachlorophényl-2-trifluoro-1,1,1-pentane-ol-2 et 25 ml d'acide sulfurique $2.10^{-2}$ molaire dans de l'acide trifluoroacétique sont chauffés au reflux pendant une durée de deux heures pour former après purification 295 mg du composé n°8 du tableau 1 ; soit un rendement de 95%.

Les structures des composés des formules I, II, III, IV, V et VI préparés dans l'ensemble des exemples précédents peuvent être caractérisées par leurs données spectrales: masse spectrale et/ou RMN (Résonance Magnétique Nucléaire de $^{1}H$, $^{19}F$, $^{13}C$).

Pour les composés de formule I, la présence du groupe trifluorométhyl et la constante de couplage JCF qui en resulte est d'une grande utilité dans l'attribution du spectre du $^{13}C$; cette donnée vient s'ajouter aux informations obtenues par les techniques et règles de routine de résonance et d'additivité. Le groupe $CF_3$ faisant fonction de marqueur interne permet de distinguer de manière certaine toutes les positions du $^{13}C$ dans les composés de formule I (sauf pour les carbones n° 9 et n° 10 que l'on peut distinguer en supposant que les substitutions sur les carbones n° 1 et n° 7 affectent le carbone n° 9).

Les spectres RMN sont enregistrés soit sur spectromètre VA-RIAN EM-360A ($^{1}H$ et $^{19}F$), soit sur un spectromètre CFT 20 ($^{13}C$) en utilisant $CDCl_3$ comme solvant et $Me_4Si$ ($^{1}H$ et $^{13}C$) ou $CFCl_3$ ($^{19}F$) comme standard interne.

Les spectres infrarouges sont réalisés sur un spectrophotomètre infrarouge type Perkins-Elmer 1240.

Caractérisation du phényl-5-trifluoro-1,1,1-pentane-one-2 de formule II :

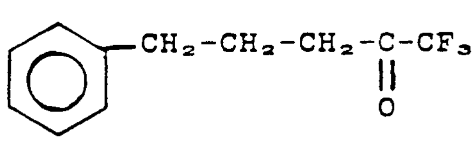

IR ($CHCl_3$) :1760 $cm^{-1}$ (c=o);1160 $cm^{-1}$;1140 $cm^{-1}$

MS m/e :216 ($M^+$);147 ($M^+CF_3$);136;118;104,91
RMN $^1H$ :2,0 (m, 3, $CH_3$);2,7 [m, 4, ($CH_2$)s];7,2(m,5, ArH)
$^{19}F$ :-80
$^{13}C$ :24,0;34,6;35,5 (3 x $CH_2$);126,4;128,5; 128,7 (ArCH);141,0 (C aromatique quaternaire);116,0 (q,J = 292 $H_z$, $CF_3$); 191,0 (q,J = 35 $H_z$, C = O)

Caractérisation du paraméthylphényl-5-trifluoro-1,1,1-pentane-one-2 de formule II :

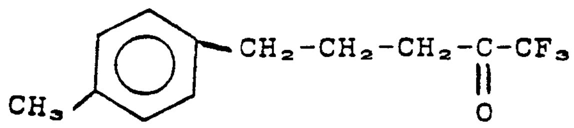

RMN $^1H$ :1,8 (t,J = 7 $H_z$, 2, $CH_2$);2,1 (s, 3, $CH_3$); 2,5 [m, 4, $(CH_2)_2$];7 (s, 4, ArH)
$^{19}F$ :-80
$^{13}C$ :21,0 ($CH_3$);24,2;34,2;35,6 (3 x $CH_2$); 1160 (q,J = 292 $H_z$, $CF_3$);128,5 :129,4 (ArCH); 136,0:138; (C aromatique quaternaire);191,0 (q,J = 35 $H_z$, C = O)

Caractérisation du paraméthoxyphényl-5-trifluoro-1,1,1-pentane-one-2 de formule II :

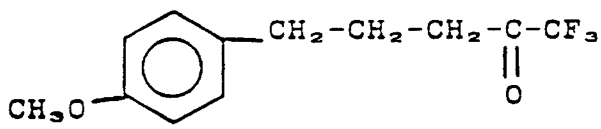

RMN $^1H$ :1,9 (m, 2, $CH_2$);2,6 [m, 4, $(CH_2)_2$];3,7 (s, 3, OMe);6,9 (q,J = 9 $H_z$, 4ArH)
$^{19}F$ :-80
$^{13}C$ :24,2;33,7;35,6 (3 x $CH_2$);55,2 ($OCH_3$); 122,0 (q,J = 286 $H_z$, $CF_3$);114,0; 129,5 (ArCH);133,0 (C aromatique quaternaire):192,0 (q,J = 34 $H_z$, C = O)

Caractérisation du parachlorophényl-5-trifluoro-1,1,1-pentane-one-2 de formule II :

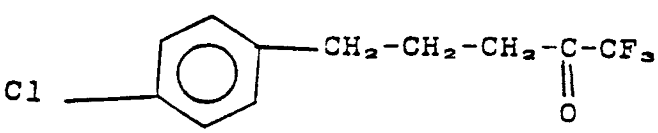

RMN $^1H$ :2,0 (m, 2, $CH_2$);2,6 [m, 4, $(CH_2)_2$];7,1 (m, 4, ArH)
$^{19}F$ :-80
$^{13}C$ :24,0;34,1;35,6 (3 x $CH_2$);116,0 (q, J = 292 $H_z$, $CF_3$);129,0; 130,0 (ArCH); 132,3;139,6 (C aromatique quaternaire): 191 (q,J = 35 $H_z$, C = O)

Caractérisation du métachlorophényl-5-trifluoro-1,1,1-pentane-one-2 de formule II :

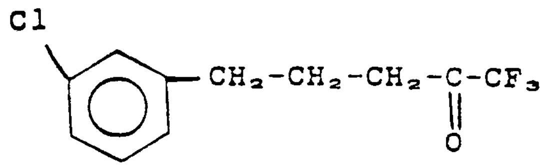

RMN $^{1}H$ :1,8 (m, 2, $CH_2$);2,4 [m, 4, $(CH_2)_2$];6,4 (m, 4, ArH)
$^{19}F$ :-80
$^{13}C$ :24,0;34,5;35,7 (3 x $CH_2$); 116,0 (q, J = 292 Hz, $CF_3$); 126,8;127,0:128,8;130,2 (ArCH);134,6; 143,3 (C aromatique quaternaire); 191,0 (q,J = 35 $H_z$, C = O)

Caractérisation du trifluorométhyl-1-dihydronaphtalène de formule V :

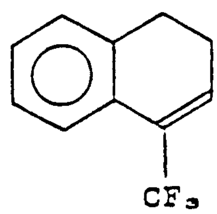

RMN $^{1}H$ : 2,50 (bm, 4H, 2 x $CH_2$);6,60 (m, 1H, C = CH);7,13 (m, 4H, aromatique)
$^{19}F$ :-64,3
$^{13}C$ : en ppm;par rapport à $Me_4Si$

| $CF_3$ | $C_1$ | $C_2$ | $C_3$ | $C_4$ | $C_5$ |
|---|---|---|---|---|---|
| 123,2 | | 132.2 | 22,5 | 27,3 | 128,2 |
| (273) | | (6,0) | | | |

| $C_6$ | $C_7$ | $C_8$ | $C_9$ | $C_{10}$ |
|---|---|---|---|---|
| 128,0 | 126,8 | 124,1 (1,5) | 128,9 | 135,9 |

entre parenthèses : constante JCF EN $H_z$

Caractérisation du trifluorométhyl-1-chloro-1-tétrahydronaphtalène 1,2,3,4 (composé n°2 du tableau 1) de formule I :

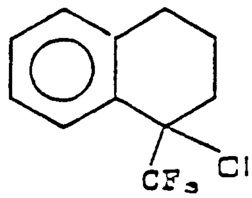

S m/e :234 ($M^+$;139 $M^+$Cl);165 ($M^+CF_3$);159;130; 128;127

Spectre de masse à haute résolution : $M^+$234, 0422
calculé pour $C_{11}H_{10}ClF_3$ 234,0423

RMN $^1H$ :2,10 (m, 2H);2,50 (m, 2H):2,80 (m, 2H); 7,25 (m, 3H, aromatique);7,80 (m, 1H, aromatique)

$^{19}F$ :-73,0

$^{13}C$ :en ppm;par rapport à $Me_4Si$

| $CF_3$ | $C_1$ | $C_2$ | $C_3$ | $C_4$ | $C_5$ |
|---|---|---|---|---|---|
| 125,5 (283) | 67,4 (29) | 35,2 | 18,9 | 29,1 | 129,3 |

| $C_6$ | $C_7$ | $C_8$ | $C_9$ | $C_{10}$ |
|---|---|---|---|---|
| 130,3 | 136,1 | 129,6 (2,5) | 131,5 | 134,9 |

entre parenthèses : constante JCF EN $H_z$

Caractérisation du trifluorométhyl-1-tétrahydronaphtalène-1, 2,3,4 (composé n°9 du tableau 1) de formule I :

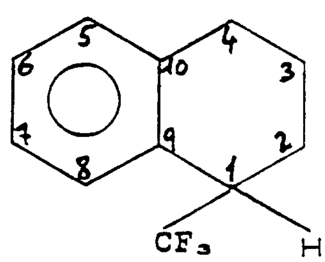

MS/me :200 ($M^+$), 131 ($M^+CF_3$);91

| | | | |
|---|---|---|---|
| calculé pour $C_{11}$,$H_{11}$,$F_3$<br>trouvé | C:66,05<br>C:66,73 | H:5,54<br>H:5,43 | F:28,50<br>F:28,5 |

RMN $^1H$ :2,0 [m, 4, $(CH_2)_2$];2,85 (M, 2, $CH_2$);3,6 (M, 1, CH aliphatique);7,1 (m, H, ArH)

$^{19}F$ :-67,0 (cl, J=6 $H_z$)

$^{13}C$ :en ppm; par rapport à $Me_4Si$

| $CF_3$ | $C_1$ | $C_2$ | $C_3$ | $C_4$ | $C_5$ |
|---|---|---|---|---|---|
| 127,7 (281) | 41,9 (25,4) | 23,1 (2,2) | 19,6 | 29,1 | 129,6 |

| $C_6$ | $C_7$ | $C_8$ | $C_9$ | $C_{10}$ |
|---|---|---|---|---|
| 125,9 | 127,8 | 130,3 (2) | 129,5 | 138,8 |

entre parenthèses : constante JCF en $H_z$

Caractérisation du trifluorométhyl-1-phényl-1-tétrahydronaphtalène-1,2,3,4 (composé n°1 du tableau 1) de formule I :

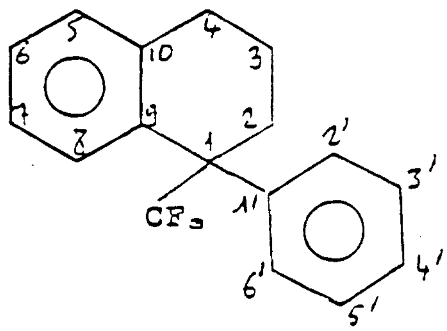

MS m/e :276 ($M^+$);207($M^+CF_3$);198;129;91

| | | | |
|---|---|---|---|
| calculé pour $C_{17}H_{15}F_3$<br>trouvé | C:73,90<br>C:73,84 | H:5,47<br>H:5,55 | F:20,63<br>F:19,57 |

RMN $^1H$ :1,80 (m, 2, $CH_2$);2,35 (m, 2, $CH_2$); 2,70 (m, 2, $CH_2$);7,30 (s, 9, ArH)
$^{19}F$ :-65,3
$^{13}C$ :en ppm; par rapport à $Me_4Si$

| $CF_3$ | $C_1$ | $C_2$ | $C_3$ | $C_4$ | $C_5$ |
|---|---|---|---|---|---|
| 128,5 (285) | 53,9 (23) | 33,9 (2,4) | 18,1 | 29,7 | 129,8 |

| $C_6$ | $C_7$ | $C_8$ | $C_9$ | $C_{10}$ |
|---|---|---|---|---|
| 127,6* | 127,1* | 130,0 (2,9) | 134,0 | 138,6 |

| $C'_1$ | $C'_2$ | $C'_3$ | $C'_4$ | $C'_5$ | $C'_6$ |
|---|---|---|---|---|---|
| 141,9 | 128,4 (2) | 128,1 | 125,9 | 128,1 | 128,4 (2) |

entre parenthèses : constante JCF en $H_z$
*indique des échanges possibles

Caractérisation du trifluorométhyl-1-paraméthylphényl-1-tétrahydronaphtalène-1,2,3,4 (composé n° 13 du tableau 1) de formule I :

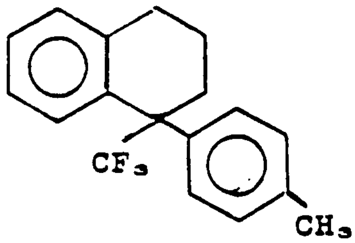

RMN $^1H$ :1,67 (m 2H);2,23 (s, 3H);2,30 (m, 2H); 2,73 (m, 2H);7,03 (s) et 7,17 (s) (8H, aromatique)
$^{19}F$ :-66,7
$^{13}C$ :en ppm;par rapport à $Me_4Si$

| $CF_3$ | $C_1$ | $C_2$ | $C_3$ | $C_4$ | $C_5$ |
|---|---|---|---|---|---|
| 129,0 (285) | 54,4 (23) | 33,8 | 18,1 | 29,7 | 129,7 |

| $C_6$ | $C_7$ | $C_8$ | $C_9$ | $C_{10}$ | $CH_3$ |
|---|---|---|---|---|---|
| 127,5 | 125,9 | 129,9 (2,7) | 134,2 | 138,6 | 20,8 |

entre parenthèses :constante JCF en $H_z$

Caractérisation du trifluorométhyl-1-paraméthoxyphényl-1-tétrahydronaphtalène-1,2,3,4 (composé n° 6 du tableau 1) de formule I :

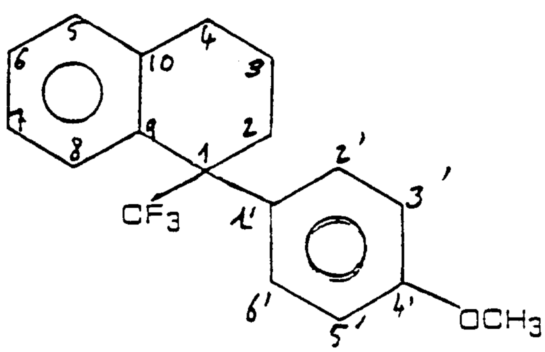

RMN $^1H$ :1,7;2,3;2,8 (m, 6, 3 x $CH_2$);3,8 (s, 3, $OCH_3$);7,0 (m, 4, ArH, H)
$^{19}F$ :-66,8
$^{13}C$ :en ppm; par rapport à $Me_4Si$

| $CF_3$ | $C_1$ | $C_2$ | $C_3$ | $C_4$ | $C_5$ |
|---|---|---|---|---|---|
| 128,6 (285) | 53,3 (23,5) | 33,6 (1,6) | 18,0 | 29,7 | 129,8 |

| $C_6$ | $C_7$ | $C_8$ | $C_9$ | $C_{10}$ |
|---|---|---|---|---|
| 125,9 | 127,6 | 129,8 (-) | 134,2 | 138,5 |

| C'$_1$ | C'$_2$ | C'$_3$ | C'$_4$ | C'$_5$ | C'$_6$ | $C(OCH_3)$ |
|---|---|---|---|---|---|---|
| 133,7 | 129,6 | 113,4 | 158,5 | 113,4 | 129,6 | 55,1 |

entre parenthèses :constante JCF EN $H_z$

Caractérisation du trifluorométhyl-1-hydroxy-1-tétrahydronaphtalène-1,2,3,4 (composé n° 11 du tableau 1) de formule I:

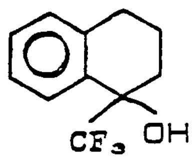

MS m/e :216 ($M^+$);198 ($M^+H_2O$);147 ($M^+CF_3$);129;91

spectre de masse haute résolution $M^+$210,0763;calculé pour $C_{11}H_{11}OF_3$ 216,0762

RMN $^1$H :1,93 (m, 4H, 2 x $CH_2$);2,70 (m, 2H); 3,30 (bs, 1H, OH);7,10 - 7,83 (m, 4H, aromatique)

$^{19}$F :-78,0

$^{13}$C :en ppm; par rapport à $Me_4Si$

| $CF_3$ | $C_1$ | $C_2$ | $C_3$ | $C_4$ | $C_5$ |
|---|---|---|---|---|---|
| 126,3 (286) | 73,0 (28,0) | 32,5 | 18,6 | 29,5 | 129,7 |

| $C_6$ | $C_7$ | $C_8$ | $C_9$ | $C_{10}$ |
|---|---|---|---|---|
| 129,0 | 126,5 | 127,4 (2,8) | 133,2 | 138,8 |

entre parenthèses : constante JCF EN $H_z$

Caractérisation du trifluorométhyl-1-chloro-1-méthyl-7-tétrahydronaphtalène (composé n° 14 du tableau 1) de formule I:

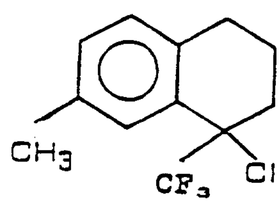

spectre de masse haute résolution $M^+$248,0579;calculé pour $C_{12}H_{12}ClF_3$ 248,0579

RMN $^1$H :2,0 (m. 2H);2,4 (5,3H + m, 2H); 2,8 (bt, 2H);7,0 - 7,6 (M, 3H, aromatique)

$^{19}$F :-73,3

$^{13}C$ :en ppm; par rapport à $Me_4Si$
$CH_3$ = 21,0

| $CF_3$ | $C_1$ | $C_2$ | $C_3$ | $C_4$ | $C_5$ |
|---|---|---|---|---|---|
| 125,6 (283) | 67,4 (29) | 35,3 | 19,0 | 29,7 | 129,4 |

| $C_6$ | $C_7$ | $C_8$ | $C_9$ | $C_{10}$ |
|---|---|---|---|---|
| 129.5 | 126.7 | 129,4 (2,5) | 131,9 | 138,1 |

entre parenthèses : constante JCF EN $H_z$

Caractérisation du trifluorométhyl-1-chloro-méthoxy-7-tétrahydronaphtalène-1,2,3,4 (composé n° 15 du tableau 1) de formule I:

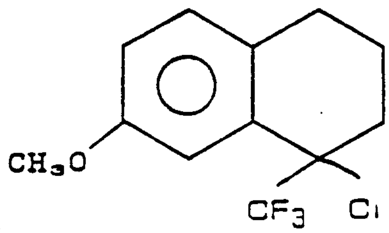

MS
spectre de masse haute résolution $M^+$264,0529; calculé pour $C_{12}H_{12}ClF_3$ 264,0529
RMN $^1H$ :2,00 (m, 2H);2,40 (m, 2H); 2,73 (m, 2H); 3,77 (s, 3H);6,67 - 7,30 (M, 3H, aromatique)
$^{19}F$ :-74,1
$^{13}C$ :en ppm; par rapport à $Me_4Si$

| $CF_3$ | $C_1$ | $C_2$ | $C_3$ | $C_4$ | $C_5$ |
|---|---|---|---|---|---|
| 125,5 (283) | 67,4 (29) | 35,2 | 19,1 | 28,8 | 130,4 |

| $C_6$ | $C_7$ | $C_8$ | $C_9$ | $C_{10}$ | $OCH_3$ |
|---|---|---|---|---|---|
| 116,3 | 158,1 | 113,9 (3,0) | 132,6 | 130,8 | 55,2 |

entre parenthèses : constante JCF EN $H_z$

Caractérisation du trifluorométhyl-1-chloro-1-chloro-7-tétranaphtalène-1,2,3,4 (composé n°16 du tableau 1) de formule I:

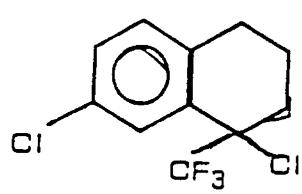

MS

spectre de masse haute résolution $M^+$268,0031;calculé pour $CH_{11}H_9Cl_2F_3$268,0033

RMN $^1H$ :2,10 (m, 2H);2,43 (m, 2H);2,83 (m, 2H) 6,86 - 7,26 (m, 2H, $H_5$ et $H_6$ aromatique) 7,67 (s, 1H, $H_8$)

$^{19}F$ :-74,7

$^{13}C$ :en ppm; par rapport à $Me_4Si$

| $CF_3$ | $C_1$ | $C_2$ | $C_3$ | $C_4$ | $C_5$ |
|---|---|---|---|---|---|
| 125,2 (283) | 66,5 (29,5) | 34,4 | 18,7 | 29,1 | 130,7 |

| $C_6$ | $C_7$ | $C_8$ | $C_9$ | $C_{10}$ |
|---|---|---|---|---|
| 129,7 | 132,3 | 129,4 (3,0) | 133,5 | 136,4 |

entre parenthèses : constante JCF EN $H_z$

Caractérisation du trifluorométhyl-1-hydroxy-1-chloro-7-tétrahydronaphtalène-1,2,3,4 (composé n° 17 du tableau 1) de formule I :

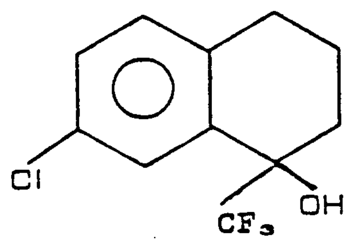

RMN $^1H$ :2,10 et 2,77 (m, x $3CH_2$) 4,5 (bm, 1H, OH);7,13 (m, 2H, H5 et H6 aromatique);7,78 (5, 1H, $H_6$)

$^{19}F$ :-78,3

$^{13}C$ :en ppm; par rapport à $Me_4Si$

| $CF_3$ | $C_1$ | $C_2$ | $C_3$ | $C_4$ | $C_5$ |
|---|---|---|---|---|---|
| 126,0 (287) | 72,8 | 32,4 | 18,5 | 28,9 | 130,7 |

| $C_6$ | $C_7$ | $C_8$ | $C_9$ | $C_{10}$ |
|---|---|---|---|---|
| 129,2 | 138,8 | 127,7 (2,5) | 137,3 | 141,0 |

entre parenthèses :constante JCF EN $H_z$

Caractérisation du trifluorométhyl-1-chloro-1-chloro-6-tétrahydronaphtalène-1,2,3,4 (composé n° 18 du tableau 1) de formule I:

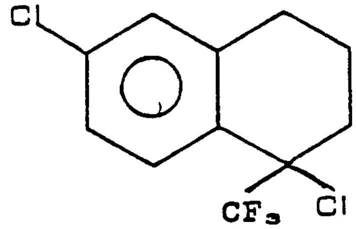

RMN $^1$H :2,03 (m,2H);2,43 (m, 2H);2,87 (m, 2H); 7,13 (m, 1H, $H_5$); 7,10 et 7,67 (m, système AB, $H_7$ et $H_8$)

$^{19}$F :-74,6

$^{13}$C :en ppm;par rapport à $Me_4Si$

| $CF_3$ | $C_1$ | $C_2$ | $C_3$ | $C_4$ | $C_5$ |
|---|---|---|---|---|---|
| 125,4 (282,5) | 66,8 (29,5) | 35,9 | 18,7 | 29,5 | 129,3 |

| $C_6$ | $C_7$ | $C_8$ | $C_9$ | $C_{10}$ |
|---|---|---|---|---|
| 135,4 | 127,1 | 131,1 (2,8) | 130,9 | 140,1 |

entre parenthèses :constante JCF EN $H_z$

Caractérisation du phényl-5-trifluoro-1,1,1-pentane-ol-2 de formule III :

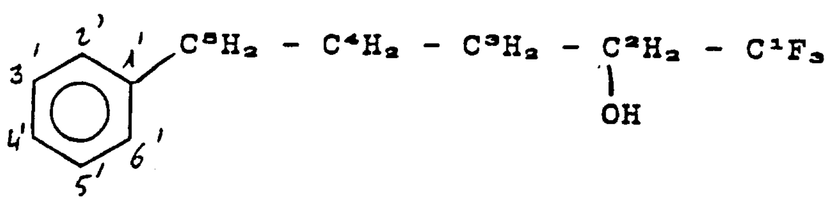

IR (film) :3350 $cm^{-1}$ ($_{OH}$);2950 $cm^{-1}$;1180 $cm^{-1}$; 1140 $cm^{-1}$

RMN $^1$H :1,7 [m, 4, $(CH_2)_2$];2,2 (s, 1, OH); 2,7 (M, 2, $CH_2$);3,9 (m, 1, $CHCF_3$); 7,2 (s 5, ArH)

$^{19}$F :-80 (d, J = 6,6 $H_z$)

$^{13}$C :en ppm;par rapport à $Me_4Si$

| $C_1(F_3)$ | $C_2(OH)$ | $C_3$ | $C_4$ | $C_5$ |
|---|---|---|---|---|
| 125,6 (282) | 70,4 (30,8) | 29,4 | 26,9 | 35,5 |

| $C'_1$ | $C'_2$ | $C'_3$ | $C'_4$ | $C'_5$ | $C'_6$ |
|---|---|---|---|---|---|
| 141,6 | 128,6 | 128,6 | 126,2 | 128,6 | 128,6 |

entre parenthèses :constante JCF EN $H_z$

Caractérisation du paraméthylphényl-5-trifluoro-1,1,1-pentane-ol-2 de formule III :

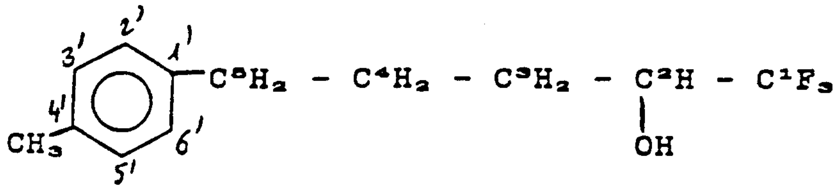

RMN $^1H$ :1,5 [m, 4, $(CH_2)_2$];2,1 (s, 3, $CH_3$); 2 5 (m, 3, $CH_2$ - OH);3,8 (m, 1, CH aliphatique);7,0 (s, 4, ArH)

$^{19}F$ :-80,5 (d, J =7$H_z$)

$^{13}C$ :en ppm; par rapport à $Me_4Si$

| $C_1(F_3)$ | $C_2$(OH) | $C_3$ | $C_4$ | $C_5$ |
|---|---|---|---|---|
| 125,5 (282) | 70,35 (30,7) | 29,2 | 27,0 | 35,5 |

| $C(CH_3)$ | $C_{1'}$ | $C_{2'}$ | $C_{3'}$ | $C_{4'}$ | $C_{5'}$ | $C_{6'}$ |
|---|---|---|---|---|---|---|
| 20,9 | 138,8 | 128,5 | 129,3 | 135,6 | 129,3 | 128,5 |

entre parenthèses : constante JCF EN $H_z$

Caractérisation du phényl-5-trifluoro-1,1,1-pentyltriflate-2 de formule IV :

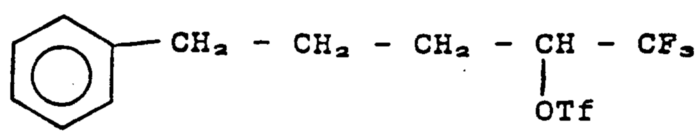

IR (film) :1440 $cm^{-1}$;1180 $cm^{-1}$;1140 $cm^{-1}$

RMN $^1H$ :1,9 [m, 4, $(CH_2)_2$];2,7 (m, 2, $CH_2$); 5,05 (m, 1, CH aliphatique);7,15 (s, 5, ArH)

$^{19}F$ :-74;-77

$^{13}C$ :26,0;28,5;35,2 (3 x $CH_2$); 82,2 (q,J = 34,2 $H_z$, CH aliphatique); 122,7 (q,J = 280 $H_z$, $CF_3$)-;126,8;128,7; 129 (ArCH); 140,8 (C aromatique quaternaire)

Caractérisation du paraméthylphényl-5-trifluoro-1,1,1-pentyltriflate-2 de formule IV :

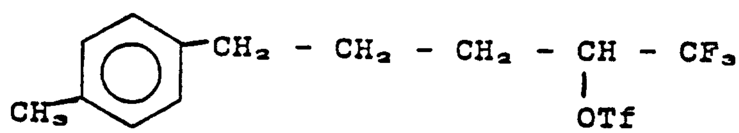

RMN $^1H$ :1,7 [m, 4, $(CH_2)_2$];2,3 (m, 2, $CH_3$); 2,5 (m, 2, $CH_2$);5,0 (m, 1, CH aliphatique); 7,0 (s, 4 ArH)

$^{19}F$ :-74,0;-76,7

$^{13}C$ :20,9 ($CH_3$);25,8;28,0;34,5 (3 x $CH_2$); 81,8 (q,J = 31,5 $H_z$, C - $CF_3$); 122 (q, J = 283 $H_z$, $CF_3$);128,5;129,4 (CH aromatique);136:137,3 (C aromatique quaternaire)

Caractérisation du paraméthoxyphényl-5-phényl-2-trifluoro-1, 1,1-pentane-ol-2 de formule III :

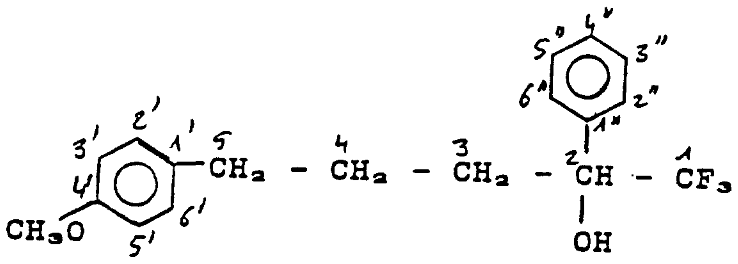

RMN $^1$H :1,9 à 2,7 [bm, 7, $(CH_2)_3$, OH]; 3,7 (s, 3, $OCH_3$);6,9;7,4 (m, 9, ArH)
$^{19}$F :-81
$^{13}$C :en ppm;par rapport à $Me_4Si$

| $C_1(F_3)$ | $C_2(OH)$ | $C_3$ | $C_4$ | $C_5$ |
|---|---|---|---|---|
| 126 (286) | 77,2 (28) | 34,9 | 24,5 | 35,5 |

| $C(OCH_3)$ | $C_1'$ | $C_2'$ | $C_3'$ | $C_4'$ | $C_5'$ | $C_6'$ |
|---|---|---|---|---|---|---|
| 55,1 | 134,0 | 129,5 | 114,0 | 158,0 | 114,0 | 129,5 |

| $C_1''$ | $C_2''$ | $C_3''$ | $C_4''$ | $C_5''$ | $C_6''$ |
|---|---|---|---|---|---|
| 137,5 | 128,3 | 128,3 | 126,9 | 128,3 | 128,3 |

entre parenthèses : constante JCF EN HZ

Caractérisation du phényl-5-paraméthoxyphényl-2-trifluoro-1, 1,1-pentane-ol-2 de formule III :

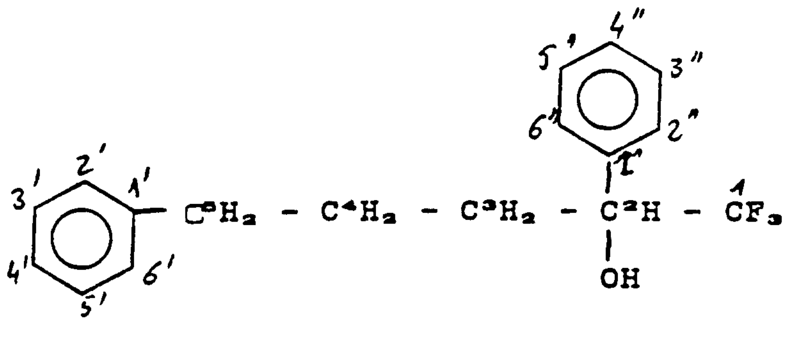

RMN $^1$H :1,6 à 2,5 [bm, 6, $(CH_2)_3$]; 2,7 (s 1, OH);3,7 (s, 3, $OCH_3$);6,8;7.1; 7,4 (m, 9, ArH)
$^{19}$F :-81
$^{13}$C :en ppm;par rapport à $Me_4Si$

| $C_1$(F3) | $C_2$(OH) | $C_3$ | $C_4$ | $C_5$ |
|---|---|---|---|---|
| 126 (286) | 77,2 (28) | 34,5 | 24,5 | 35,7 |

| $C_1'$ | $C_2'$ | $C_3'$ | $C_4'$ | $C_5'$ | $C_6'$ |
|---|---|---|---|---|---|
| 141 | 128,5 | 128,5 | 126,1 | 128,5 | 128,5 |

| C($OCH_3$) | $C_1''$ | $C_2''$ | $C_3''$ | $C_4''$ | $C_5''$ | $C_6''$ |
|---|---|---|---|---|---|---|
| 55,1 | 128,7 | 127,9 | 113,8 | 159,5 | 113,8 | 127,9 |

entre parenthèses : constante JCF en HZ

Caractérisation du métachlorophényl-5-phényl-2-trifuoro-1,1, 1-pentane-ol-2 de formule III :

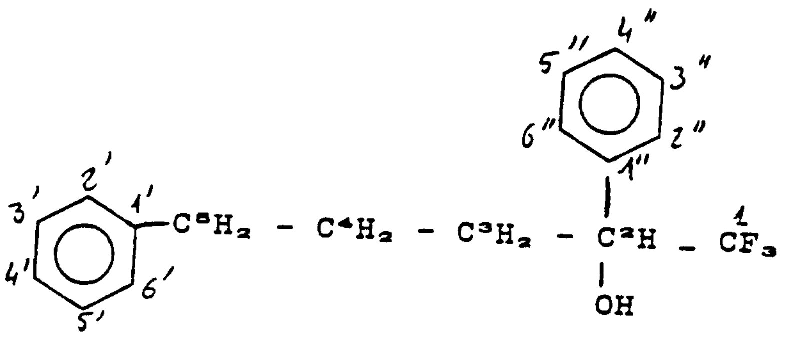

RMN $^1$H :1,7 à 2,5 [bm, 7, $(CH_2)_3$, OH]; 7,1 - 7,4 (m, 9, ArH)
$^{19}$F :-81
$^{13}$C :en ppm; par rapport à $Me_4Si$

| $C_1(F_3)$ | $C_2$(OH) | $C_3$ | $C_4$ | $C_5$ |
|---|---|---|---|---|
| 126 (286) | 77,5 (28) | 34,5 | 23,7 | 35,2 |

| $C_1'$ | $C_2'$ | $C_3'$ | $C_4'$ | $C_5'$ | $C_6'$ |
|---|---|---|---|---|---|
| 143,7 | 126,3 | 129,7 | 126,7 | 134,2 | 128,5 |

| $C_1''$ | $C_2''$ | $C_3''$ | $C_4''$ | $C_5''$ | $C_6''$ |
|---|---|---|---|---|---|
| 136,3 | 128,5 | 128,5 | 126,3 | 128,5 | 128,5 |

entre parenthèses : constante JCF en HZ

Caractérisation du phényl-5-métachlorophényl-2-trifluoro-1,1, 1-pentane-ol-2 de formule III :

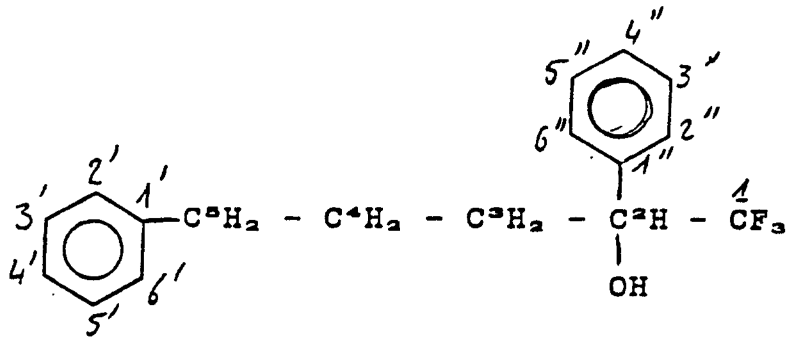

RMN $^1H$ :1,7 à 2,5 [bm, 7, $(CH_2)_3$, OH] 7,3 (m, 9, ArH)

$^{19}F$ :-81

$^{13}C$ :en ppm; par rapport à $Me_4Si$

| $C_1(F_3)$ | $C_2(OH)$ | $C_3$ | $C_4$ | $C_5$ |
|---|---|---|---|---|
| 125,3 (286) | 76,4 (28) | 34,2 | 23,6 | 35,2 |

| $C_1'$ | $C_2'$ | $C_3'$ | $C_4'$ | $C_5'$ | $C_6'$ |
|---|---|---|---|---|---|
| 141 | 128,1 | 128,2 | 126,7 | 128,2 | 128,1 |

| $C_1''$ | $C_2''$ | $C_3''$ | $C_4''$ | $C_5''$ | $C_6''$ |
|---|---|---|---|---|---|
| 138,4 | 128,3 | 134,3 | 125,8 | 129,3 | 124,3 |

entre parenthèses : constante JCF en HZ

Caractérisation du trifluorométhyl-1-méthyl-7-tétrahydronaphtalène-1,2,3,4 (composé n° 10 du tableau 1) de formule I:

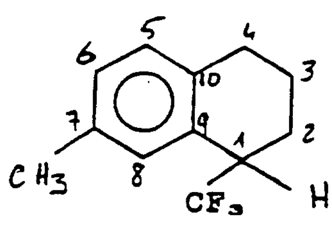

RMN $^1H$ :2,0 [bm, 4 $(CH_2)_2$];2,3 (s, 3, $CH_3$); 2,8 (m, 2, $CH_2$);3,5 (m, 1, H aliphatique); 7,1 (m, 3, ArH)

$^{19}F$ :-67,0 (d,J = 6 $H_z$)

$^{13}C$ :en ppm; par rapport à $Me_4Si$

| $CF_3$ | $C(CH_3)$ | $C_1$ | $C_2$ | $C_3$ | $C_4$ | $C_5$ |
|---|---|---|---|---|---|---|
| 127,6 (281) | 21,04 | 41,7 (25,7) | 23,1 (-) | 19,6 | 28,6 | 129,3 |

| $C_6$ | $C_7$ | $C_8$ | $C_9$ | $C_{10}$ |
|---|---|---|---|---|
| 128,6 | 135,6 | 130,7 (-) | - | 136,0 |

entre parenthèses : constante JCF en $H_z$

Caractérisation du trifluorométhyl-1-méthyl-7-phényl-1-tétrahydronaphtalène-1,2,3,4 (composé n°2 du tableau 1) de formule I :

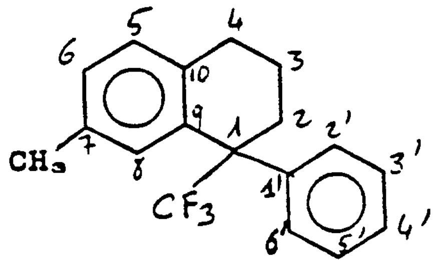

RMN $^1H$ :1,4 à 2,4 [bm, 4 $(CH_2)_2$];2,1 (s, 3, $CH_3$); 2,7 (m, 2, $CH_2$);7,1 (s, 8, ArH)
$^{19}F$ :-65,8
$^{13}C$ :en ppm; par rapport à $Me_4Si$

| $CF_3$ | $C(CH_3)$ | $C_1$ | $C_2$ | $C_3$ | $C_4$ | $C_5$ |
|---|---|---|---|---|---|---|
| 128,4 (284) | 21,1 | 54,0 (23,3) | 34,0 (1,3) | 18,1 | 29,2 | 129,5 |

| $C_6$ | $C_7$ | $C_8$ | $C_9$ | $C_{10}$ |
|---|---|---|---|---|
| 128,0 | 135,5 | 130,2 (2,6) | 133,7 | 135,2 |

| $C_{1'}$ | $C_{2'}$ | $C_{3'}$ | $C_{4'}$ | $C_{5'}$ | $C_{6'}$ |
|---|---|---|---|---|---|
| 142,0 | 128,5 (2,2) | 128,0 | 127,0 | 128,0 | 128,5 (2,2) |

entre parenthèses : constante JCF en $H_z$

Caractérisation du trifluorométhyl-1-méthoxy-7-phényl-1-tétrahydronaphtalène-1,2,3,4 (composé n° 3 du tableau 1) de formule I :

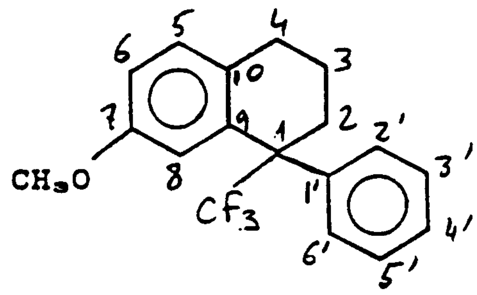

RMN $^1H$ :1,7 à 2,7 [m, 6 $(CH_2)_3$];3,65 (s, 3, $OCH_3$); 6,8 (m, 3, ArH);7,2 (s, 5, ArH)
$^{19}F$ :-65,0
$^{13}C$ :en ppm; par rapport à $Me_4Si$

| $CF_3$ | $C(OCH_3)$ | $C_1$ | $C_2$ | $C_3$ | $C_4$ | $C_5$ |
|---|---|---|---|---|---|---|
| 128,5 (285) | 55,2 | 54,2 (23,7) | 33,9 (-) | 18,3 | 28,9 | 130,5 |

| $C_6$ | $C_7$ | $C_8$ | $C_9$ | $C_{10}$ |
|---|---|---|---|---|
| 114,1 | 157,6 | 114,8 (3) | 134,8 | 130,8 |

| $C_1'$ | $C_2'$ | $C_3'$ | $C_4'$ | $C_5'$ | $C_6'$ |
|---|---|---|---|---|---|
| 141,8 | 128,5 (1,5) | 128,1 | 127,2 | 128,1 | 128,5 (1,5) |

entre parenthèses : constante JCF en $H_z$

Caractérisation du trifluorométhyl-1-chloro-7-phényl-1-tétrahydronaphtalène-1,2,3,4 (composé n° 4 du tableau 1) de formule I :

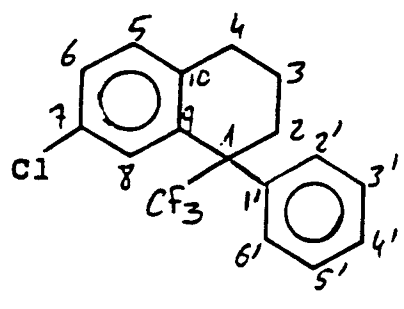

RMN $^1H$ :1,8;2,3;2,8 (3 x $CH_2$);7,2 (m, 8, ArH)
$^{19}F$ :-66,7
$^{13}C$ :en ppm; par rapport à $Me_4Si$

| $CF_3$ | $C_1$ | $C_2$ | $C_3$ | $C_4$ | $C_5$ |
|---|---|---|---|---|---|
| 128,1 (285) | 54,0 (23,7) | 33,6 (1,7) | 17,9 | 29,1 | 131,0 |

| $C_6$ | $C_7$ | $C_8$ | $C_9$ | $C_{10}$ |
|---|---|---|---|---|
| 127,9 | 131,6 | 129,9 (2,9) | 135,8 | 137,2 |

| $C_1'$ | $C_2'$ | $C_3'$ | $C_4'$ | $C_5'$ | $C_6'$ |
|---|---|---|---|---|---|
| 141,2 | 128,3* | 127,9* | 127,4 | 127,9 | 128,3 |

entre parenthèses : constante JCF en $H_z$
*indique des échanges possibles

Caractérisation du trifluorométhyl-1-chloro-6-phényl-1-tétrahydronaphtalène-1,2,3,4 (composé n°5 du tableau 1) de formule I :

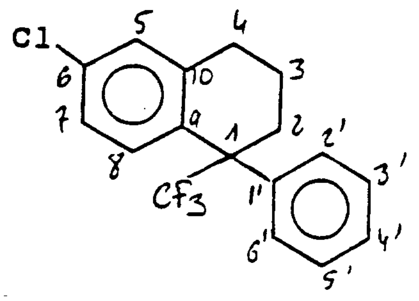

RMN $^1$H :1,7;2,3;2,7 (m, 6, 3 x $CH_2$); 7,13 (s, 8, ArH)
$^{19}$F :-66
$^{13}$C :en ppm; par rapport à $Me_4Si$

| $CF_3$ | $C_1$ | $C_2$ | $C_3$ | $C_4$ | $C_5$ |
|---|---|---|---|---|---|
| 128,2 (285) | 53,7 (24,2) | 33,8 (2) | 17,9 | 29,6 | 129,5 |

| $C_6$ | $C_7$ | $C_8$ | $C_9$ | $C_{10}$ |
|---|---|---|---|---|
| 133,6 | 127,4 | 131,5 (3) | 132,7 | 140,8* |

| $C_1'$ | $C_2'$ | $C_3'$ | $C_4'$ | $C_5'$ | $C_6'$ |
|---|---|---|---|---|---|
| 141,5 | 128,3* | 127,4* | 126,3 | 127,4* | 128,3* |

entre parenthèses : constante JCF en $H_z$
*indique des échanges possibles

Caractérisation du trifluorométhyl-1-parachlorophényl-1-tétrahydronaphtalène (composé n° 7 du tableau 1) de formule I :

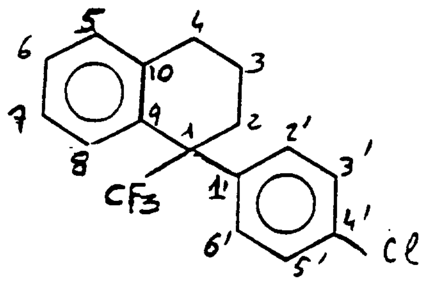

RMN $^1H$ :1,7;2,3;2,8 (m, 6, 3 x $CH_2$); 7,2 (m, 8, ArH)
$^{19}F$ :-65,3
$^{13}C$ :en ppm; par rapport à $Me_4Si$

| $CF_3$ | $C_1$ | $C_2$ | $C_3$ | $C_4$ | $C_5$ |
|---|---|---|---|---|---|
| 128,3 (285) | 53,7 (24) | 33,9 (1,5) | 18,1 | 29,6 | 129,9 |

| $C_6$ | $C_7$ | $C_8$ | $C_9$ | $C_{10}$ |
|---|---|---|---|---|
| 126,2 | 127,9 | 133,4 (3,9) | 133,5 | 138,6 |

| $C_1'$ | $C_2'$ | $C_3'$ | $C_4'$ | $C_5'$ | $C_6'$ |
|---|---|---|---|---|---|
| 140,5 | 129,9 | 128,3 | 133,3 | 128,3 | 129,9 |

entre parenthèses : constante JCF en $H_z$

Caractérisation du trifluorométhyl-1-métachlorophényl-1-tétrahydronaphtalène-1,2,3,4 (composé n° 8 du tableau 1) de formule I :

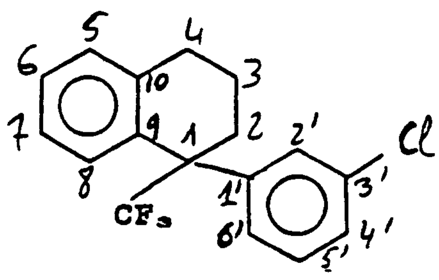

RMN $^1H$ :1,5;2,2;2,8 (m, 6, 3 x $CH_2$); 7,2 (m, 8, ArH)
$^{19}F$ :-66.7
$^{13}C$ :en ppm;par rapport à $Me_4Si$

| $CF_3$ | $C_1$ | $C_2$ | $C_3$ | $C_4$ | $C_5$ |
|---|---|---|---|---|---|
| 128,2 (286) | 53,9 (24) | 33,9 (2) | 18,1 | 29,6 | 129,9 |

| $C_6$ | $C_7$ | $C_8$ | $C_9$ | $C_{10}$ |
|---|---|---|---|---|
| 126,2 | 127,9 | 129,8 (3) | 133,2 | 138,6 |

| $C_1'$ | $C_2'$ | $C_3'$ | $C_4'$ | $C_5'$ | $C_6'$ |
|---|---|---|---|---|---|
| 144,1 | 128,8 | 134,3 | 126,2 | 129,4 | 126,8 |

entre parenthèses : constante JCF en $H_z$

**Revendications**

**1.** Composés répondant à la formule générale :

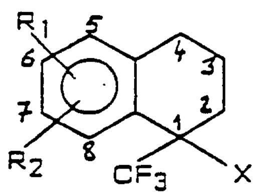

I

dans laquelle :

- X représente un atome d'hydrogène, un halogène, un groupe hydroxy, un groupe alcoxy en $C_1$ à $C_8$, ou un noyau aromatique éventuellement substitué ;
- $R_1$ représente un atome d'hydrogène ou un halogène, un groupe hydroxy, un groupe alcoxy en $C_1$ à $C_8$, un groupe alkyle linéaire ou ramifié en $C_1$ à $C_4$ éventuellement halogéné, occupant l'une des positions 5, 6 ou 7, ou encore un groupe méthylène-dioxy occupant les positions 5 et 6 ou 6 et 7, et
- $R_2$ représente un atome d'hydrogène ou un halogène, un groupe hydroxy, un groupe alcoxy en $C_1$ à $C_8$, un groupe alkyle linéaire ou ramifié en $C_1$ à $C_4$ éventuellement halogéné, occupant l'une des autres positions 5, 6 ou 7.

**2.** Composés selon la revendication 1, caractérisés en ce que dans la formule I, X représente un noyau aromatique, notamment phényl, naphtyl, ou thienyl, pouvant porter un à deux substituants choisis parmi halogène, hydroxy, alcoxy en $C_1$ à $C_4$, trifluorométhyl ou alkyle linéaire ou ramifié en $C_1$ à $C_4$éventuellement halogéné.

**3.** Composés selon la revendication 2, caractérisés en ce que dans la formule I, X représente un noyau phényl pouvant porter un à deux atomes de chlore ou de fluor.

**4.** Le trifluorométhyl-1-phényl-1-tétrahydronaphtaléne-1,2,3,4.

**5.** Le trifluorométhyl-1-phényl-1-méthyl-7-tétrahydronaphtalène-1,2,3,4.

**6.** Le trifluorométhyl-1-phényl-1-méthoxy-7-tétrahydronaphtalène-1,2,3,4.

**7.** Le trifluorométhyl-1-phényl-1-chloro-7-tétrahydronaphtalène-1,2,3,4.

8. Le trifluorométhyl-1-phényl-1-chloro-6-tétrahydronaphtalène-1,2,3,4.

9. Le trifluorométhyl-1-orthométhoxyphényl-1-tétrahydronaphtalène-1,2,3,4.

10. Le trifluorométhyl-1-métaméthoxyphényl-1-tétrahydronaphtalène-1,2,3,4.

11. Le trifluorométhyl-1-paraméthoxyphényl-1-tétrahydronaphtalène-1,2,3,4.

12. Le trifluorométhyl-1-parachlorophényl-1-tétrahydronaphtalène-1,2,3,4.

13. Le trifluorométhyl-1-orthochlorophényl-1-tétrahydronaphtalène-1,2,3,4.

14. Le trifluorométhyl-1-métachlorophényl-1-tétrahydronaphtalène-1,2,3,4.

15. Le trifluorométhyl-1-dichlorométaparaphényl-1-tétrahydronaphtalène-1,2,3,4.

16. Le trifluorométhyl-1-tétrahydronaphtalène-1,2,3,4.

17. Le trifluorométhyl-1-méthyl-7-tétrahydronaphtaléne-1,2,3, 4.

18. Le trifluorométhyl-1-chloro-1-méthyl-7-tétrahydronaphtalène-1,2,3,4.

19. Le trifluorométhyl-1-hydroxy-1-chloro-7-tétrahydronaphtalène-1,2,3,4.

20. Le trifluorométhyl-1-chloro-1-chloro-6-tétrahydronaphtalène-1,2,3,4.

21. Procédé de préparation d'un composé conforme à l'une quelconque des revendications 1 à 20, caractérisé en ce que l'on réalise une réaction de Friedel et Crafts intramoléculaire d'un aryl-5-trifluoro-1,1,1-pentane-one-2 de formule II

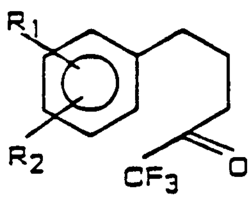

dans laquelle $R_1$ et $R_2$ ont la même signification que dans la formule I, dans un solvant et en présence d'un acide de Lewis.

22. Procédé de préparation d'un composé de formule I dans laquelle X représente un noyau aromatique éventuellement substitué selon la revendication 21, caractérisé en ce que l'on utilise comme solvant un hydrocarbure aromatique correspondant au radical X.

23. Procédé selon l'une quelconque des revendications 21 ou 22, caractérisé en ce que l'acide de Lewis est un acide de Lewis aluminique ou $TiCl_4$.

24. Procédé de préparation d'un composé conforme à l'une quelconque des revendications 2 à 20, caractérisé en ce que l'on effectue une solvolyse d'un dérivé d'un aryl-5-trifluoro-1,1,1-pentane-ol-2 de formule III

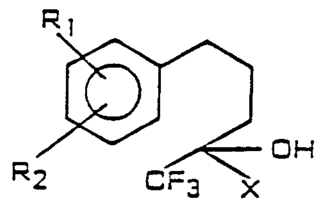

dans laquelle $R_1$ et $R_2$ ont la même signification que dans la formule I et X représente un noyau aromatique éventuellement substitué, pour donner le composé de formule I correspondant.

**25.** Procédé selon la revendication 24, caractérisé en ce que la solvolyse d'un dérivé de formule III est effectuée avec un acide protique tel que l'acide trifluoroacétique.

**26.** Procédé selon la revendication 25, caractérisé en ce que la solvolyse d'un dérivé de formule III est effectuée dans un autoclave maintenu à une température comprise entre 120 ° C et 180 ° C pendant une durée comprise entre soixante minutes et deux cent quarante minutes.

**27.** Procédé selon la revendication 24, caractérisé en ce que la solvolyse d'un dérivé de formule III est effectuée avec un acide protique, tel que l'acide sulfurique, dans un solvant tel que l'acide trifluoroacétique, le dichlorométhane, le benzène ou le toluène.

**28.** Procédé selon la revendication 27 caractérisé en ce que la solvolyse d'un dérivé de formule III est effectuée au reflux pendant une durée inférieure à douze heures.

**29.** Procédé selon la revendication 24, caractérisé en ce que la solvolyse d'un dérivé de formule III est effectuée avec un acide de Lewis dans un solvant tel que le dichlorométhane, le le benzène ou le toluène.

**30.** Procédé selon la revendication 29, caractérisé en ce que la solvolyse est effectuée à température ambiante, pendant une durée comprise entre deux heures et cinq heures.

**31.** Procédé de préparation d'un composé de formule I selon la revendication 1, dans laquelle X représente un atome d'hydrogène, caractérisé en ce que l'on effectue une solvolyse d'un dérivé d'un aryl-5-trifluoro-1,1,1 pentane-ol-2 protégé de formule IV

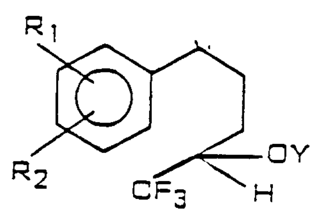

dans laquelle :
$R_1$ et $R_2$ ont la méme signification que dans la formule I et OY représente un groupe partant, notamment un group tosyle, un groupe triflique, un groupe méthane sulfonique, pour obtenir le composé de formule I correspondant.

**32.** Procédé selon la formule 31, caractérisé en ce que l'on effectue la solvolyse d'un dérivé de formule IV dans laquelle OY représente un groupe triflique.

**33.** Application d'un composé conforme à l'une quelconque des revendications 1 à 20, pour la synthèse de l'amino trifluorométhyl tétraline correspondante.

## Claims

1. Compounds corresponding to the general formula:

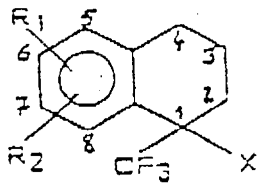

I

in which:

- X represents an hydrogen atom, a halogen, a hydroxy group, a $C_1$ to $C_8$ alkoxy group, or an optionally substituted aromatic ring;
- $R_1$ represents a hydrogen atom or a halogen, a hydroxy group, a $C_1$ to $C_8$ alkoxy group, an optionally halogenated linear or branched $C_1$ to $C_4$ alkyl group, occupying one of the positions 5, 6 or 7, or also a methylene-dioxy group occupying the positions 5 and 6 or 6 and 7, and
- $R_2$ represents a hydrogen atom or a halogen, a hydroxy group, a $C_1$ to $C_8$ alkoxy group, an optionally halogenated linear or branched $C_1$ to $C_4$ alkyl group, occupying one of the other positions 5, 6 or 7.

2. Compounds according to claim 1, characterised in that in the formula I, X represents an aromatic ring, in particular phenyl, naphthyl or thienyl, able to carry one to two substituents chosen from halogen, hydroxy, $C_1$ to $C_4$ alkoxy, trifluoromethyl or optionally halogenated $C_1$ to $C_4$ linear or branched alkyl.

3. Compounds according to claim 2, characterised in that in the formula I, X represents a phenyl ring able to carry one to two chlorine or fluorine atoms.

4. 1-Trifluoromethyl-1-phenyl-1,2,3,4-tetrahydronaphthalene.

5. 1-Trifluoromethyl-1-phenyl-7-methyl-1,2,3,4-tetrahydronaphthalene.

6. 1-Trifluoromethyl-1-phenyl-7-methoxy-1,2,3,4-tetrahydronaphthalene.

7. 1-Trifluoromethyl-1-phenyl-7-chloro-1,2,3,4-tetrahydronaphthalene.

8. 1-Trifluoromethyl-1-phenyl-6-chloro-1,2,3,4-tetahydronaphthalene.

9. 1-Trifluoromethyl-1-ortho-methoxyphenyl-1,2,3,4-tetrahydronaphthalene.

10. 1-Trifluoronethyl-1-meta-methoxyphenyl-1,2,3,4-tetrahydronaphthalene.

11. 1-Trifluoromethyl-1-para-methoxyphenyl-1,2,3,4-tetrahydronaphthalene.

12. 1-Trifluoromethyl-1-para-chlorophenyl-1,2,3,4-tetrahydronaphthalene.

13. 1-Trifluoromethyl-1-ortho-chlorophenyl-1,2,3,4-tetrahydronaphthalene.

14. 1-Trifluoromethyl-1-meta-chlorophenyl-1,2,3,4-tetrahydronaphthalene.

15. 1-Trifluoromethyl-1-dichloro-meta-paraphenyl-1,2,3,4-tetrahydronapnthalene.

16. 1-Trifluoromethyl-1,2,3,4-tetrahydronaphthalene.

17. 1-Trifluoromethyl-7-methyl-1,2,3,4-tetrahydronaphthalene.

**18.** 1-Trifluoromethyl-1-chloro-7-methyl-1,2,3,4-tetrahydronaphthalene.

**19.** 1-Trifluoromethyl-1-hydroxy-7-chloro-1,2,3,4-tetrahydronaphthalene.

**20.** 1-Trifluoromethyl-1-chloro-6-chloro-1,2,3,4-tetrahydronaphthalene.

**21.** Process for preparing a compound according to one of claims 1 to 20, characterised in that an intramolecular Friedel Crafts reaction is carried out using 5-aryl-1,1,1-trifluoropentane-2-one of the formula II

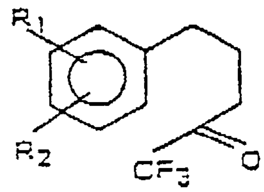

in which $R_1$ and $R_2$ have the same meaning as in formula I, in a solvent and in the presence of a Lewis acid.

**22.** Process for preparing a compound of the formula I, in which X represents an optionally substituted aromatic ring according to claim 21, characterised in that an aromatic hydrocarbon corresponding to the radical X is used as the solvent.

**23.** Process according to any one of claims 21 or 22, characterised in that the Lewis acid is an aluminic Lewis acid or $TiCl_4$.

**24.** Process for preparing a compound corresponding to any one of claims 2 to 20, characterized in that solvolysis of a derivative of a 5-aryl-1,1,1-trifluoropentane-2-ol of the formula III

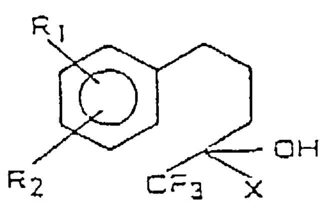

in which $R_1$ and $R_2$ have the same meaning as in formula I and X represents an optionally substituted aromatic ring, is carried out to give the corresponding compound of formula I.

**25.** Process according to claim 24, characterised in that solvolysis of a derivative of formula III is carried out using a protic acid, such as trifluoroacetic acid.

**26.** Process according to claim 25, characterised in that solvolysis of a derivative of formula III is carried out in an autoclave maintained at a temperature between 120°C and 180°C and for between sixty minutes and two hundred and forty minutes.

**27.** Process according to claim 24, characterised in that solvolysis of a derivative of formula III is carried out using a protic acid, such as sulphuric acid, in a solvent, such as trifluoroacetic acid, dichloromethane, benzene or toluene.

**28.** Process according to claim 27, characterised in that solvolysis of a derivative of formula III is carried out under reflux for a period of less than 12 hours.

**29.** Process according to claim 24, characterised in that solvolysis of a derivative of formula III is carried out using a Lewis acid in a solvent, such as dichloromethane, benzene or toluene.

**30.** Process according to claim 29, characterised in that solvolysis is carried out at ambient temperature for a period between two hours and five hours.

**31.** Process for preparing a compound of formula I according to claim 1, in which X represents a hydrogen atom, characterised in that solvolysis of a derivative of a 5-aryl-1,1,1-trifluoropentane-2-ol derived from formula IV

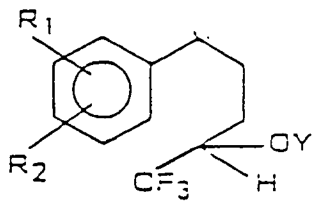

in which:
$R_1$ and $R_2$ have the same meaning as in formula I and OY represents a leaving group, in particular a tosyl group, a triflic group, a methane sulphonic group, is carried out to give the corresponding compound of formula I.

**32.** Process according to formula 31, characterised in that solvolysis of a derivative of formula IV, in which OY represents a triflic group, is carried out.

**33.** Use of a compound according to any one of claims 1 to 20, for synthesising the corresponding aminotrifluoromethyl tetraline.

**Patentansprüche**

**1.** Verbindungen der allgemeinen Formel:

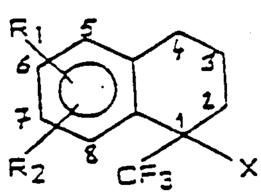

I

worin bedeuten:

- X ein Wasserstoffatom, ein Halogentom, eine Hydroxygruppe, eine $C_1$-$C_8$-Alkoxygruppe oder einen gegebenenfalls substituierten aromatischen Kern;
- $R_1$ ein Wasserstoffatom oder ein Halogentom, eine Hydroxygruppe, eine $C_1$-$C_8$-Alkoxygruppe, eine gegebenenfalls halogenierte, lineare oder verzweigte $C_1$-$C_4$-Alkylgruppe, die eine der Positionen 5, 6 oder 7 besetzt, oder auch eine Methylendioxy-Gruppe, welche die Positionen 5 und 6 oder 6 und 7 besetzt, und
- $R_2$ ein Wasserstoffatom oder ein Halogenatom, eine Hydroxygruppe, eine $C_1$-$C_8$-Alkoxygrupe, eine gegebenenfalls halogenierte lineare oder verzweigte $C_1$-$C_4$-Alkylgruppe,die eine der übrigen Positionen 5, 6 oder 7 besetzt.

**2.** Verbindungen nach Anspruch 1, dadurch gekennzeichnet, daß in der Formel (I) X steht für einen aromatischen Kern, insbesondere Phenyl-, Naphthyl- oder Thienyl-Kern, der 1 bis 2 Substituenten, ausgewählt aus Halogen, Hydroxy, $C_1$-$C_4$-Alkoxy, Trifluoromethyl oder gegebenenfalls halogeniertem linearem oder verzweigtem $C_1$-$C_4$-Alkyl, tragen kann.

3. Verbindungen nach Anspruch 2, dadurch gekennzeichnet, daß in der Formel (I) X steht für einen Phenylkern, der 1 bis 2 Chlor- oder Fluoratome tragen kann.

4. 1-Trifluoromethyl-1-phenyl-1,2,3,4-tetrahydronaphthalin.

5. 1-Trifluoromethyl-1-phenyl-7-methyl-1,2,3,4-tetrahydronaphthalin.

6. 1-Trifluoromethyl-1-phenyl-7-methoxy-1,2,3,4-tetrahydronaphthalin.

7. 1-Trifluoromethyl-1-phenyl-7-chloro-1,2,3,4-tetrahydronaphthalin.

8. 1-Trifluoromethyl-1-phenyl-6-chloro-1,2,3,4-tetrahydronaphthalin.

9. 1-Trifluoromethyl-1-o-methoxyphenyl-1,2,3,4-tetrahydronaphthalin.

10. 1-Trifluoromethyl-1-m-methoxyphenyl-1,2,3,4-tetrahydronaphthalin.

11. 1-Trifluoromethyl-1-p-methoxyphenyl-1,2,3,4-tetrahydronaphthalin.

12. 1-Trifluoromethyl-1-p-chlorophenyl-1,2,3,4-tetrahydronaphthalin.

13. 1-Trifluoromethyl-1-o-chloroyphenyl-1,2,3,4-tetrahydronaphthalin.

14. 1-Trifluoromethyl-1-m-chlorophenyl-1,2,3,4-tetrahydronaphthalin.

15. 1-Trifluoromethyl-1-dichloromethaparaphenyl-1,2,3,4-tetrahydronaphthalin.

16. 1-Trifluoromethyl-1,2,3,4-tetrahydronaphthalin.

17. 1-Trifluoromethyl-7-methyl-1,2,3,4-tetrahydronaphthalin.

18. 1-Trifluoromethyl-1-chloro-7-methyl-1,2,3,4-tetrahydronaphthalin.

19. 1-Trifluoromethyl-1-hydroxy-7-chloro-1,2,3,4-tetrahydronaphthalin.

20. 1-Trifluoromethyl-1-chloro-6-chloro-1,2,3,4-tetrahydronaphthalin.

21. Verfahren zur Herstellung einer Verbindung nach einem der Ansprüche 1 bis 20, dadurch gekennzeichnet, daß man eine intramolekulare Friedel-Crafts-Reaktion eines 5-Aryl-1,1,1-trifluoro-pentan-2-ons der Formel

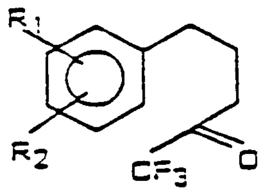

II

worin $R_1$ und $R_2$ die gleichen Bedeutungen haben wie in der Formel (I) angegeben, in einem Lösungsmittel und in Gegenwart einer Lewis-Säure durchführt.

22. Verfahren zur Herstellung einer Verbindung der Formel (I), in der X steht für einen gegebenenfalls substituierten aromatischen Kern, nach Anspruch 21, dadurch gekennzeichnet, daß man als Lösungsmittel einen dem Rest X entsprechenden aromatischen Kohlenwasserstoff verwendet.

**23.** Verfahren nach einem der Ansprüche 21 oder 22, dadurch gekennzeichnet, daß es sich bei der Lewis-Säure um eine aluminiumhaltige Lewis-Säure oder $TiCl_4$ handelt.

**24.** Verfahren zur Herstellung einer Verbindung nach einem der Ansprüche 2 bis 20, dadurch gekennzeichnet, daß man eine Solvolyse eines Derivats eines 5-Aryl-1,1,1-trifluoro-pentan-2-ols der Formel

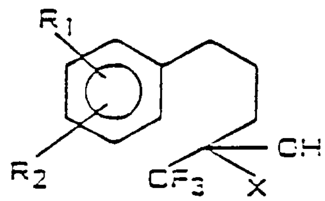

III

worin $R_1$ und $R_2$ die gleichen Bedeutungen haben wie in der Formel (I) angegeben und X für einen gegebenenfalls substituierten aromatischen Kern steht, durchführt zur Bildung der entsprechenden Verbindung der Formel (I).

**25.** Verfahren nach Anspruch 24, dadurch gekennzeichnet, daß die Solvolyse eines Derivats der Formel (III) mit einer protischen Säure, wie Trifluoroessigsäure, durchgeführt wird.

**26.** Verfahren nach Anspruch 25, dadurch gekennzeichnet, daß die Solvolyse eines Derivats der Formel (III) in einem bei einer Temperatur zwischen 120 und 180°C gehaltenen Autoklaven während einer Zeitspanne zwischen 60 und 240 min durchgeführt wird.

**27.** Verfahren nach Anspruch 24, dadurch gekennzeichnet, daß die Solvolyse eines Derivats der Formel (III) mit einer protischen Säure wie Schwefelsäure in einem Lösungsmittel, wie Trifluoressigsäure, Dichlormethan, Benzol oder Toluol, durchgeführt wird.

**28.** Verfahren nach Anspruch 27, dadurch gekennzeichnet, daß die Solvolyse eines Derivats der Formel (III) unter Rückfluß während einer Zeitspanne von weniger als 12 h durchgeführt wird.

**29.** Verfahren nach Anspruch 24, dadurch gekennzeichnet, daß die Solvolyse eines Derivats der Formel (III) mit einer Lewis-Säure in einem Lösungsmittel, wie Dichlormethan, Benzol oder Toluol, durchgeführt wird.

**30.** Verfahren nach Anspruch 29, dadurch gekennzeichnet, daß die Solvolyse bei Umgebungstemperatur während einer Zeitspanne zwischen 2 und 5 h durchgeführt wird.

**31.** Verfahren zur Herstellung einer Verbindung der Formel (I) nach Anspruch 1, in der X ein Wasserstoffatom bedeutet, dadurch gekennzeichnet, daß man eine Solvolyse eines Derivats eines geschützten 5-Aryl-1,1,1-trifluoro-pentan-2-ols der Formel

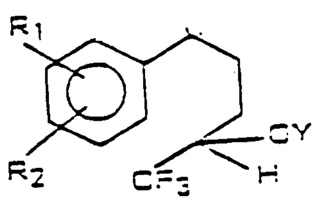

IV

worin $R_1$ und $R_2$ die gleichen Bedeutungen wie in der Formel (I) haben und OY eine abspaltbare Gruppe, insbesondere eine Tosyl-Gruppe, eine Trifluoromethylgruppe, eine Methansulfonsäuregruppe darstellt, durchführt zur Herstellung der entsprechenden Verbindung der Formel (I).

**32.** Verfahren nach Anspruch 31, dadurch gekennzeichnet, daß man die Solvolyse eines Derivats der Formel (IV) durchführt, in der OY eine Trifluoromethylgruppe darstellt.

**33.** Verwendung einer Verbindung nach einem der Ansprüche 1 bis 20 für die Synthese des entsprechenden Trifluoromethylaminotetralins.

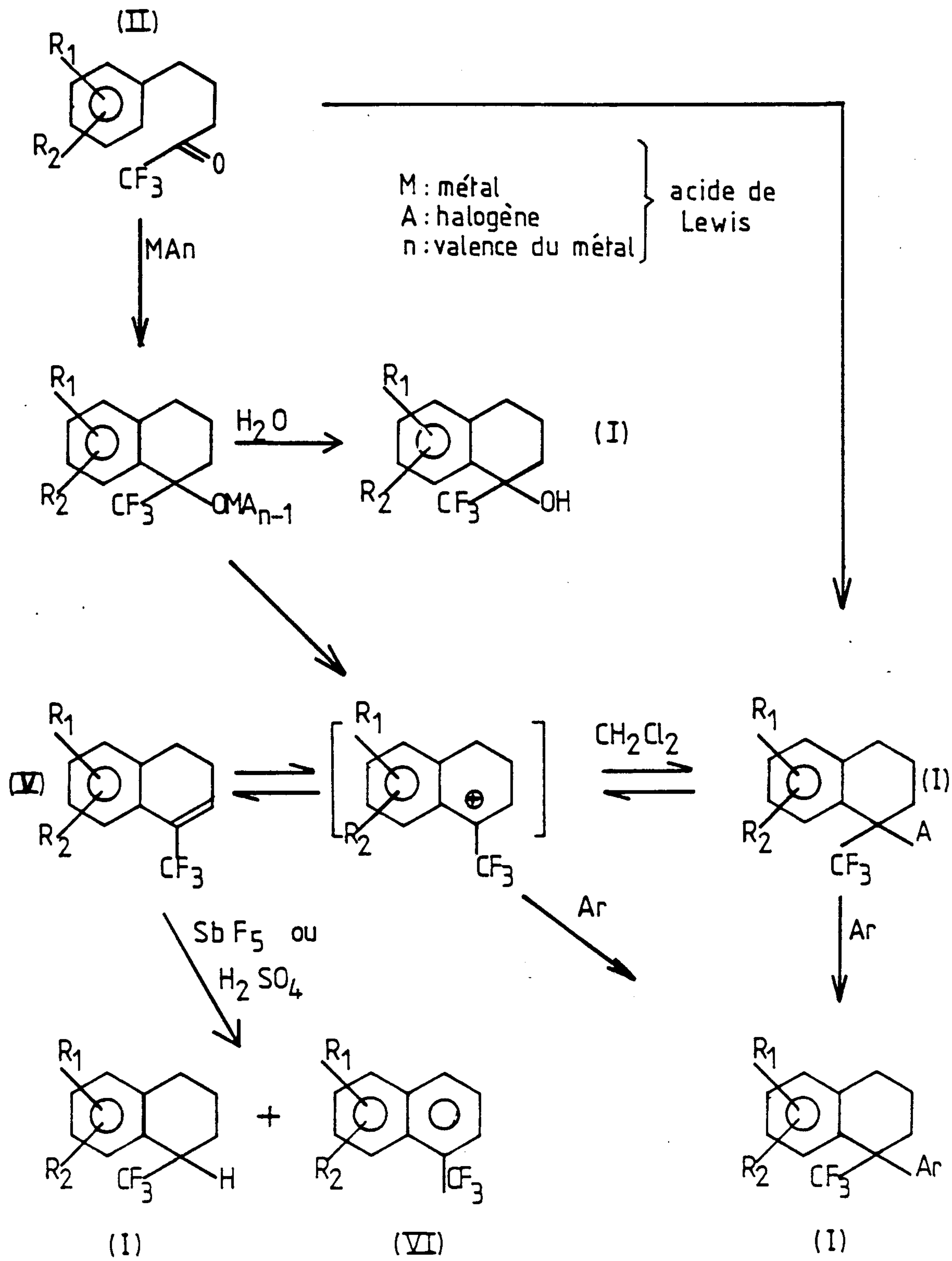
(II)
MAn
M : métal
A : halogène
n : valence du métal
acide de Lewis
$H_2O$
(I)
(V)
$CH_2Cl_2$
(I)
$SbF_5$ ou $H_2SO_4$
Ar
Ar
(I)
(VI)
(I)